(19) Europäisches Patentamt
European Patent Office
Office européen des brevets

(11) **EP 3 849 575 B1**

(12) **FASCICULE DE BREVET EUROPEEN**

(45) Date de publication et mention de la délivrance du brevet:
**03.07.2024 Bulletin 2024/27**

(21) Numéro de dépôt: **19786381.4**

(22) Date de dépôt: **10.09.2019**

(51) Classification Internationale des Brevets (IPC):
**A61K 36/31** (2006.01)    **A61K 36/481** (2006.01)
**A61P 3/00** (2006.01)    **A61P 3/04** (2006.01)
**A61P 3/06** (2006.01)    **A61P 3/08** (2006.01)
**A61P 3/10** (2006.01)

(52) Classification Coopérative des Brevets (CPC):
(C-Sets disponibles)
**A61P 3/00; A61K 36/31; A61K 36/481; A61P 3/04; A61P 3/06; A61P 3/08; A61P 3/10**    (Cont.)

(86) Numéro de dépôt international:
**PCT/FR2019/052092**

(87) Numéro de publication internationale:
**WO 2020/053519 (19.03.2020 Gazette 2020/12)**

(54) **EXTRAITS DE MORICANDIA POUR LEUR UTILISATION DANS LA PRÉVENTION ET LE TRAITEMENT DES MALADIES MÉTABOLIQUES ET POUR LA PRÉVENTION, LE CONTRÔLE ET LE TRAITEMENT DE LA PRISE DE POIDS**

MORICANDIA-EXTRAKTE ZUR VORBEUGUNG UND BEHANDLUNG VON STOFFWECHSELERKRANKUNGEN SOWIE ZUR VORBEUGUNG, KONTROLLE UND BEHANDLUNG VON GEWICHTSZUNAHME

MORICANDIA EXTRACTS FOR USE IN THE PREVENTION AND TREATMENT OF METABOLIC DISEASES AND FOR THE PREVENTION, CONTROL AND TREATMENT OF WEIGHT GAIN

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **10.09.2018 FR 1858094**

(43) Date de publication de la demande:
**21.07.2021 Bulletin 2021/29**

(73) Titulaires:
• **Université de Bourgogne**
  **21000 Dijon (FR)**
• **Centre national de la recherche scientifique**
  **75016 Paris (FR)**
• **Institut national de recherche pour l'agriculture, l'alimentation et l'environnement**
  **75007 Paris (FR)**
• **Université de Monastir**
  **Monastir, 5000 (TN)**

(72) Inventeurs:
• **ABED, Basma**
  **Tataouine (TN)**
• **LELOUP, Corinne**
  **21380 Messigny et Vantoux (FR)**

(74) Mandataire: **Icosa**
  **83 avenue Denfert-Rochereau**
  **75014 Paris (FR)**

(56) Documents cités:
**US-B2- 8 946 283**

• **MARIANGELA MARRELLI ET AL: "Phytochemical and Biological Profile of Moricandia arvensis (L.) DC.: An Inhibitor of Pancreatic Lipase", MOLECULES, vol. 23, no. 11, 31 octobre 2018 (2018-10-31), page 2829, XP055591282, DOI: 10.3390/molecules23112829**

EP 3 849 575 B1

- Anonymous: "Flavonoids from the Genus Astragalus: Phytochemistry and Biological Activity", , 1 janvier 2016 (2016-01-01), XP055591301, Extrait de l'Internet: URL:https://www.ncbi.nlm.nih.gov/pmc/articles/PMC4791984/?report=printable [extrait le 2019-05-23]
- SKANDRANI I ET AL: "Moricandia arvensis extracts protect against DNA damage, mutagenesis in bacteria system and scavenge the superoxide anion", TOXICOLOGY IN VITRO, ELSEVIER SCIENCE, GB, vol. 23, no. 1, 1 février 2009 (2009-02-01), pages 166-175, XP025870254, ISSN: 0887-2333, DOI: 10.1016/J.TIV.2008.10.010 [extrait le 2008-10-30]
- INES SKANDRANI ET AL: "Leaf and root extracts of Moricandia arvensis protect against DNA damage in human lymphoblast cell K562 and enhance antioxidant activity", ENVIRONMENTAL TOXICOLOGY AND PHARMACOLOGY, vol. 30, no. 1, 1 juillet 2010 (2010-07-01), pages 61-67, XP055591322, NL ISSN: 1382-6689, DOI: 10.1016/j.etap.2010.03.014
- MOSTAFA KHALIL ET AL: "Anti-Helicobacter pylori, anti-diabetic and cytotoxicity activity of biosynthesized gold nanoparticles using Moricandia nitens water extract", EGYPTIAN JOURNAL OF CHEMISTRY, vol. 0, no. 0, 10 juin 2018 (2018-06-10), pages 0-0, XP055591323, DOI: 10.21608/ejchem.2018.3744.1318

(52) Classification Coopérative des Brevets (CPC): (Cont.)

C-Sets
A61K 36/31, A61K 2300/00;
A61K 36/481, A61K 2300/00

**Description**

**DOMAINE DE L'INVENTION**

La présente invention se rapporte à une composition comprenant un extrait de *Moricandia*, pour son utilisation dans la prévention et/ou le traitement des maladies métaboliques liées à une intolérance au glucose et/ou à une résistance à l'insuline. Elle se rapporte aussi à une composition comprenant un extrait de *Moricandia*, pour son utilisation dans la prévention de la prise de poids, notamment de masse grasse.

**ÉTAT DE LA TECHNIQUE**

Les maladies métaboliques et leurs conséquences sont parmi les causes de décès les plus courantes dans le monde. Les maladies cardiaques, comorbidité du diabète, est une cause première de mortalité - elles représentent 32% de l'ensemble des décès - devant le cancer et les maladies respiratoires chroniques, ce fléau mondial touche plus de 100 millions de personnes et les experts prévoient qu'il causera plus de 25 millions de morts en 2030, contre 17,5 millions en 2005. Aujourd'hui, le syndrome métabolique est tellement répandu qu'on estime qu'aux États-Unis, un adulte sur quatre en est atteint; en Europe, il toucherait 15% des adultes.

Le diabète de type 2, ou diabète non insulino-dépendant, est la forme de diabète la plus fréquente puisqu'il touche environ 90% des personnes diabétiques. La maladie concerne toutes les classes d'âge mais sa fréquence augmente avec l'âge. À titre d'exemple, la fréquence de la maladie atteint 25% au-delà de 65 ans aux États-Unis ; et au-delà de 75 ans en France. Au niveau mondial, la fréquence du diabète chez les adultes est passée de 4,7% en 1980 à 8,5% en 2015. La mortalité associée de manière directe au diabète est estimée à 1,6 million de morts par an. De plus, des études récentes ont montré que le diabète s'accompagne, dans 60% des cas, d'une apparition de nombreux autres facteurs de risque métabolique tels que l'hypertension, le surpoids voire l'obésité, et la dyslipidémie. La recherche de traitement pour le diabète de type 2 reste donc aujourd'hui un enjeu majeur.

Le diabète est une maladie métabolique caractérisée par un excès chronique de sucre dans le sang (hyperglycémie), correspondant à une glycémie (taux de glucose dans le sang) à jeun supérieure à 1,26 g/L. Dans un contexte non-pathologique, la glycémie est maintenue constante par :

(i) l'absorption de glucides provenant de l'alimentation au niveau des intestins ;
(ii) la mise en réserve du glucose, via la sécrétion d'insuline par le pancréas endocrine qui stimule l'absorption de glucose par les tissus dit « sensibles à l'insuline », principalement les muscles, les tissus adipeux et le foie ; et
(iii) la libération de glucose dans le sang, principalement par le foie, en période interprandiale.

La résistance à l'insuline, *i.e.*, lorsque la réponse des cellules à l'insuline est diminuée, notamment suite à l'apparition d'un état inflammatoire de bas grade, lié le plus souvent à une masse adipeuse viscérale trop développée, s'accompagne également d'un défaut d'inhibition de la production hépatique de glucose. La capture du glucose étant diminuée, cet état conduit à l'apparition du diabète de type 2. Le pancréas endocrine (composé des îlots de Langerhans) peut compenser cette résistance dans un premier temps en sécrétant plus d'insuline, mais suite à cette sollicitation excessive conduisant à l'épuisement du pancréas, une déficience en insuline (*i.e.*, un défaut de synthèse d'insuline par les cellules β des îlots de Langerhans) s'installe. Cette déficience requiert alors une insulinothérapie.

D'autres pathologies sont associées à une dérégulation de la glycémie, telles que le syndrome métabolique, les maladies liées à la résistance à l'insuline ou liée à une déficience en insuline, à l'intolérance au glucose, l'hyperglycémie, l'obésité, la dyslipidémie, l'hypercholestérolémie, l'hypertriglycéridémie et le stress oxydatif.

Le traitement des maladies associées à une dérégulation de la glycémie, au-delà de recommandations diététiques et d'augmentation de l'activité physique, repose sur l'injection d'insuline ou sur l'utilisation d'agents hypoglycémiants tels que les sulfonylurées, qui stimulent la sécrétion d'insuline ; les biguanides (comme la metformine, la phenformine et la buformine), qui améliorent la sensibilité à l'insuline ; les inhibiteurs d'α-glucosidase, qui ralentissent la digestion du saccharose et des carbohydrates complexes ; et les thiazolidinediones qui, en combinaison avec une insulinothérapie, diminuent la résistance à l'insuline.

Cependant, ces approches thérapeutiques sont associées avec de nombreux effets indésirables. Par exemple, l'administration de sulfonylurées peut conduire à une hypoglycémie, des maladies rénales et hépatiques, augmenter le risque d'accident cardiovasculaire, déclencher des réactions dermatologiques indésirables, des vertiges et des maux de tête. L'acidose lactique et l'augmentation du risque d'accident cardiovasculaire font également partie des effets secondaires des biguanides. Les inhibiteurs d'α-glucosidase peuvent conduire à une hypoglycémie et à des troubles gastro-intestinaux. Enfin, parmi les inconvénients fréquemment associés à l'utilisation des thiazolidinediones figurent la dépendance de leur efficacité à la présence d'insuline, l'induction d'une rétention d'eau, la diminution du taux de globules rouges, l'induction de maux de tête, ainsi que les risques d'hépatotoxicité et de décompensation cardiaque.

Ces effets indésirables ont d'ailleurs abouti à l'interdiction en Europe et au retrait aux États-Unis en Mars 2000 de la troglitazone (Rezulin®); et, après une limitation des indications de la rosiglitazone (Avandia®) et de la pioglitazone (Actos®), à la suspension de leur AMM en Europe en 2010 et 2011 respectivement. La demande de brevet US 8 946 283 porte sur l'utilisation du composé phytochimique indole-3-carbinol contenu en grande quantité dans les plantes du genre Brassica pour le traitement de l'obésité, la dyslipidémie ou le diabète.

Dans ce contexte, l'utilisation de plantes médicinales apparait comme potentiellement avantageuse. En effet, du fait de leur potentielle action sur des cibles multiples, leur utilisation permet d'obtenir un effet thérapeutique tout en limitant le risque d'effets secondaires.

Ici, les Inventeurs ont identifié deux espèces de plantes, *Moricandia arvensis* et *Astragalus armatus* et démontré leurs effets préventifs et curatifs pour le diabète de type 2 (DT2). Ces effets bénéfiques suggèrent par ailleurs leur potentielle utilisation dans la prévention et/ou le traitement d'autres pathologies associées à une dérégulation de la glycémie.

*Moricandia arvensis* appartient à la famille des brassicacées et est distribuée dans la région méditerranéenne, particulièrement dans les régions désertiques du Nord de l'Afrique. Cette plante, parfois appelée « moricandie des champs » ou « chou des champs », est couramment décrite pour son utilisation dans le traitement de la syphilis, des maux de tête et du scorbut, et présente des propriétés antioxydantes et de protection des dommages à l'ADN (Skandrani et al., 2010. Food Chem Toxicol. 48(2):710-5; Skandrani et al., 2007. Drug Chem Toxicol. 30(4):361-82; Braham et al., 2005. J Nat Prod. 68(4):517-22).

*Astragalus armatus* ou « astragale vulnérant » appartient au genre des astragales, qui comprend 2500 espèces, et à la famille des fabacées. Cette plante se retrouve dans le Nord de l'Afrique, particulièrement au Maghreb. *Astragalus armatus* est couramment utilisée dans le traitement du rhume, de l'asthénie, des diarrhées, de la grippe, de l'asthme et de l'arthrose. Les graines et l'écorce sont utilisées plus spécifiquement dans le traitement de la douleur, de fièvres, de la constipation, des morsures de serpent et de scorpion. Cette plante présente par ailleurs des propriétés antioxydantes, anti-complément, anticholinestérase, antibactérienne et pro-phagocytaire.

De manière inattendue, les Inventeurs ont observé qu'un traitement avec un extrait de *Moricandia arvensis* et/ou *d'Astragalus armatus* permet de prévenir l'hyperglycémie, la résistance à l'insuline, l'intolérance au glucose, la prise de poids et l'augmentation du ratio masse grasse/masse maigre, dans un modèle de développement du DT2. D'autre part, le traitement avec un extrait de *Moricandia arvensis* et/ou *d'Astragalus armatus* améliore la plupart de ces paramètres dans un modèle pré-clinique de diabète de type 2. Les Inventeurs ont également mis en évidence le fait que *Moricandia arvensis* et *Astragalus armatus* ont un effet protecteur de la capacité sécrétoire des cellules pancréatiques.

Dans leur ensemble, ces résultats indiquent une nouvelle utilisation de *Moricandia arvensis* et *d'Astragalus armatus* dans la prévention et/ou le traitement des maladies cardiométaboliques, de préférence des maladies métaboliques liées à une intolérance au glucose et/ou à une résistance à l'insuline.

## RÉSUMÉ

La présente invention se rapporte à une composition comprenant une décoction de feuilles de *Moricandia arvensis*, optionnellement lyophilisée, pour son utilisation dans la prévention et/ou le traitement d'une maladie métabolique sélectionnée dans le groupe comprenant le diabète, la résistance à l'insuline, l'intolérance au glucose, et l'hyperglycémie..

Dans un mode de réalisation, la maladie métabolique est le diabète de type 2.

Dans un mode de réalisation, la composition comprend en outre un extrait *d'Astragalus armatus.*

Dans un mode de réalisation, l'extrait *d'Astragalus* est une décoction et/ou une macération *d'Astragalus armatus.*

Dans un mode de réalisation, l'extrait d'Astragalus est une macération de racines *d'Astragalus armatus*, de préférence une macération méthanolique de racines *d'Astragalus armatus*, optionnellement lyophilisé.

La présente invention se rapporte également à un alicament comprenant une décoction de feuilles de *Moricandia arvensis*, optionnellement lyophilisée.

La présente invention se rapporte également à une méthode non-thérapeutique :

- de prévention de la prise de poids, de préférence de prévention de la prise de masse grasse chez un sujet ;
- de contrôle de la prise de poids, de préférence de contrôle de la prise de masse grasse chez un sujet ; ou
- de stimulation de la perte de poids, de préférence de stimulation de la perte de masse grasse chez un sujet ;

comprenant l'administration audit sujet de l'alicament selon la présente invention.

La présente invention se rapporte enfin à composition comprenant une décoction de feuilles de *Moricandia arvensis*, optionnellement lyophilisée, pour son utilisation dans :

- la prévention de la prise de poids chez un sujet, de préférence la prévention de la prise de masse grasse chez un sujet ;
- le contrôle de la prise de poids chez un sujet, de préférence le contrôle de la prise de masse grasse chez un sujet ; ou
- la stimulation de la perte de poids chez un sujet, de préférence la stimulation de la perte de masse grasse chez un sujet.

Dans un mode de réalisation, la composition comprend en outre un extrait d'*Astragalus armatus*, de préférence une macération de racines *d'Astragalus armatus*, de préférence une macération méthanolique de racines *d'Astragalus armatus*, optionnellement lyophilisé.

## DÉFINITIONS

Dans la présente invention, les termes ci-dessous sont définis de la manière suivante :
Le terme « *Astragalus* », tel qu'utilisé ici, désigne un genre de plante appartenant à la famille des fabacées. Le genre inclus plus de 2500 espèces différentes, lesquelles sont cataloguées et indexées en ligne, par exemple dans la base de données Species 2000 (Roskov et al., Eds. (2018). Species 2000 & ITIS Catalogue of Life, 30th June 2018. Digital resource at www.catalogueoflife.org/col. Species 2000: Naturalis, Leiden, the Netherlands. ISSN 2405-8858). Une sélection d'espèces représentatives du genre *Astragalus* comprend, sans y être limité, *Astragalus alopecuroides*, *Astragalus alopecurus, Astragalus alpinus, Astragalus amphioxys, Astragalus armatus, Astragalus australis, Astragalus austriacus, Astragalus baionensis, Astragalus boeticus, Astragalus cicer, Astragalus crassicarpus, Astragalus crenatus, Astragalus frigidus, Astragalus glycyphyllos, Astragalus hamosus, Astragalus hypoglottis, Astragalus lentiginosus, Astragalus membranaceus, Astragalus monspessulanus, Astragalus onobrychis, Astragalus penduliflorus, Astragalus propinquus, Astragalus sempervirens* et *Astragalus tragacantha*. Tel qu'utilisé ici, le terme « *Astragalus* » couvre cependant l'ensemble des espèces d'Astragalus connues à ce jour, ainsi que les espèces nouvelles non-découvertes à ce jour.

Le terme « *Astragalus armatus* », tel qu'utilisé ici, désigne une espèce de plantes appartenant à la famille des Fabacées et principalement distribuée au Maghreb, plus particulièrement dans les zones présahariennes. L'espèce inclus, sans y être limité, les sous-espèces et variétés suivantes : *Astragalus armatus armatus, Astragalus armatus libyens, Astragalus armatus numidicus, Astragalus armatus tragacanthoides* et *Astragalus armatus tumidus.*

Le terme « **athérosclérose** », tel qu'utilisé ici, se réfère à une maladie caractérisée par l'obstruction des artères de moyen et gros calibre (aorte et ses branches, artères coronaires, artères cérébrales, artères des membres inférieurs) consécutive à l'apparition de plaques d'athérome. L'athérosclérose est notamment responsable des coronaropathies et des maladies cérébro-vasculaires ischémiques.

L'expression « **consiste essentiellement en** », telle qu'utilisé ici en référence à une composition, se réfère à ces compositions dans lesquelles le composé les constituant, *e.g.*, un extrait, est le seul agent ayant une activité biologique.

Le terme « **coronaropathie** », également appelée « **maladie coronarienne** » ou « **insuffisance coronarienne** », tel qu'utilisé ici, se réfère à un groupe de maladies causées par une ischémie myocardique (irrigation insuffisante du myocarde en raison de la formation d'athéromes dans les artères coronaires). La coronaropathie regroupe ainsi l'angine de poitrine, l'infarctus du myocarde et la mort cardiaque subite.

Le terme « **décoction »** tel qu'utilisé ici, désigne une technique d'extraction aqueuse consistant en l'incubation d'un matériel végétal dans l'eau chaude, préférentiellement dans l'eau bouillante, plus préférentiellement en maintenant l'ébullition pendant une durée supérieure à environ 5 minutes, de préférence pendant une durée supérieure à 6, 7, 8, 9, 10, 11, 12, 12 ou 14 minutes, plus préférentiellement pendant une durée supérieure à 15 minutes. Dans un mode de réalisation, la décoction est réalisée à partir d'un matériel végétal « frais », *i.e.*, n'ayant subi aucune étape de séchage préalable. Dans un mode de réalisation, la décoction est réalisée à partir d'un matériel végétal « sec », également défini ici comme « **matière végétale sèche** », *i.e.*, ayant subi une étape de séchage préalable.

Le terme « **diabète** », tel qu'utilisé ici, désigne les maladies métaboliques caractérisées par un excès chronique de sucre (glucose) dans le sang, aussi appelées « diabète sucré ». L'un des critères utilisés pour le diagnostic du diabète est une glycémie à jeun supérieure à 1,26 g/L de sang (soit environ 7 mmol/L de sang). On distingue plusieurs types de diabète :

- le « **diabète de type 1** », aussi appelé « **diabète inné** », est caractérisé par la disparition chez l'enfant ou chez le jeune adulte des cellules β des îlots de Langerhans du pancréas, productrices d'insuline (maladie auto-immune) ; et
- le « **diabète de type 2** », aussi appelé « **diabète non insulinodépendant** », est caractérisé par l'apparition progressive d'une résistance à l'insuline (*i.e.*, lorsque la réponse des cellules sensibles à l'insuline est diminuée). La résistance à l'insuline se manifeste par la diminution de la capture du glucose par les tissus adipeux et les muscles et la diminution de l'inhibition de la production hépatique de glucose. À un stade plus avancé, le diabète de type 2 peut conduire à une déficience en insuline, *i.e.*, un défaut de synthèse d'insuline par les cellules du pancréas endocrine.

Le terme « **dyslipidémie** », tel qu'utilisé ici, se réfère à un état, pathologique si chronique, dans lequel une quantité anormalement élevée ou au contraire, anormalement diminuée, de lipides (cholestérol, triglycérides, phospholipides ou acides gras libres) circule dans le sang. La dyslipidémie regroupe ainsi d'un côté l'hyperlipidémie (dont plus spécifiquement l'hypercholestérolémie et l'hypertriglycéridémie) et l'hypolipidémie (dont plus spécifiquement l'hypocholestérolémie).

Le terme « **environ** », lorsqu'il précède une valeur chiffrée, signifie plus ou moins 10% de ladite valeur.

Le terme « **extrait** », tel qu'utilisé ici, désigne un matériau qui a été retiré d'une plante, ou d'une ou plusieurs parties de celle-ci telle que, de manière non-limitative, les fleurs, les fruits, les graines, les feuilles, les racines, les feuilles et/ou la tige. Tel que cela est connu de l'homme du métier, un extrait peut être soit brut, soit raffiné à un degré choisi afin d'isoler des substances ou agents actifs spécifiés. Un extrait peut se présenter sous différentes formes telles qu'un jus, une décoction, une infusion, un produit de fermentation, une teinture, un macérat huileux, un macérat hydroalcoolique glycériné, une purée ou une poudre. De nombreuses techniques d'extraction, connue de l'homme du métier peuvent être employées seules ou en combinaison : la déshydratation, le séchage à l'air, le séchage au micro-ondes, le séchage au four, la lyophilisation, la filtration, l'évaporation, le broyage la macération, la percolation, l'infusion, la décoction, l'extraction par Soxhlet, l'extraction continue à chaud, l'extraction assistée par micro-ondes, l'extraction assistée par ultrasons, l'extraction accélérée par solvant, l'extraction par fluide supercritique, l'extraction aqueuse, l'extraction alcoolique (notamment méthanolique ou éthanolique), la distillation à la vapeur, l'enfleurage, la digestion enzymatique et l'ensemble des techniques apparentées.

Le terme « **extrait sec** », tel qu'utilisé ici, désigne le produit obtenu à l'issue d'une étape d'extraction et dans lequel le solvant d'extraction a été retiré. Les techniques permettant l'obtention d'un extrait sec sont bien connues de l'homme du métier, et incluent, sans y être limité, la « **lyophilisation** » et l'évaporation.

Le terme « **hypercholestérolémie** », tel qu'utilisé ici, se réfère à un état, pathologique si chronique, dans lequel une quantité excessive de cholestérol circule dans le sang. Cette quantité correspond à une cholestérolémie totale > 2,0 g/L avant 30 ans et > 2,5 g/L après 30 ans.

Le terme « **hyperglycémie** », tel qu'utilisé ici, se réfère à un état, pathologique si chronique, dans lequel une quantité excessive de glucose circule dans le sang. Selon l'Association Française des Diabétique, cette quantité correspond à une glycémie > 1,26 g/L à jeun et > 2,00 g/L le reste du temps.

Le terme « **hypertension artérielle** », tel qu'utilisé ici, se réfère à une pathologie définie par une pression artérielle trop élevée. On parle communément d'hypertension artérielle pour une pression artérielle systolique > 140 mmHg et une pression artérielle diastolique > 90 mmHg.

Le terme « **hypertriglycéridémie** », tel qu'utilisé ici, se réfère à un état, pathologique si chronique, dans lequel une quantité excessive de triglycérides circule dans le sang. Cette quantité correspond à une triglycéridémie > 1,5 g/L, soit > 1,7 mmol/L.

Le terme « **intolérance au glucose** », parfois aussi appelé « **tolérance abaissée au glucose** », tel qu'utilisé ici, se réfère à une maladie souvent considérée comme transitoire vers le diabète de type 2. L'intolérance au glucose peut cependant précéder l'apparition d'un diabète de type 2 pendant plusieurs années. L'intolérance au glucose est définie par l'Organisation Mondiale de la Santé par un niveau de glucose sanguin de 6,1 à 6,9 mmol/L à jeun ; et/ou 7,8 à 11,1 mmol/L deux heures après l'ingestion de 75 g de glucose.

Le terme « **lyophilisation** », tel qu'utilisé ici, désigne l'élimination, par sublimation, du solvant d'une composition préalablement congelée, permettant ainsi d'obtenir un « **extrait sec** ». Le solvant sublimé est généralement de l'eau, mais peut également être un alcool.

Le terme « **macération** », tel qu'utilisé ici, désigne une technique consistant à incuber un matériel végétal dans un solvant, de préférence dans un solvant à température ambiante ou froid (*i.e.*, à environ 20-25°C ou moins), afin d'en extraire les composés solubles. Des exemples de solvant pouvant être utilisés pour une macération incluent, sans y être limité, une solution alcoolique (telles que, *e.g.*, une solution d'éthanol, d'isopropanol ou de méthanol), de l'eau, de la saumure ou une huile.

Le terme « **maladies cérébro-vasculaires** », tel qu'utilisé ici, se réfère à un ensemble des maladies se manifestant par l'apparition brutale d'un déficit neurologique dû à des lésions cérébrales d'origine vasculaire. En particulier, les maladies cérébro-vasculaires hémorragiques sont dues à la rupture d'un vaisseau sanguin tandis que les maladies cérébro-vasculaires ischémiques sont consécutives à l'obstruction d'une artère cérébrale (voir : athérosclérose).

Les termes « **maladies liées à la résistance à ou une déficience en insuline** » et **« maladies liées à une intolérance au glucose** », tels qu'utilisés ici, regroupent plusieurs maladies métaboliques dont le diabète de type 2, le syndrome métabolique, mais également les maladies cardiovasculaires (Ford, 2005. Diabètes Care. 28(7): 1769-78), la stéatose hépatique non alcoolique (Bugianesi et al., 2010. Curr Pharm Des. 16(17):1941-51), le syndrome des ovaires poly-kystiques (SOPK) (Diamanti-Kandarakis, 2006. Endocrine. 30(1): 13-7), la maladie d'Alzheimer (Watson & Craft, 2003. CNS Drugs. 17(1):27-45) et le cancer (Arcidiacono et al., 2012. Exp Diabètes Res. 2012:789174).

Le terme « **matériel végétal** », tel qu'utilisé ici, désigne le produit utilisé pour la production d'un extrait végétal utile pour la mise en oeuvre de la présente invention. Un matériel végétal peut consister en une plante entière, avec ou sans ses racines, ou d'une ou plusieurs parties d'une plante, comme par exemple, les fleurs, les fruits, les graines, le pollen, les racines, les tiges, les feuilles et/ou les écorces. Dans un mode de réalisation, le matériel végétal est « frais », *i.e.*, il n'a subi aucune étape de séchage préalable. Dans un mode de réalisation, le matériel végétal est sec (aussi définit ici comme « **matière végétale sèche** »), *i.e.*, il a subi une étape de séchage préalable.

Les termes « **matière végétale sèche** » et « **matériel végétal sec** », tel qu'utilisé ici, désignent un matériel végétal ayant subi une étape de séchage, soit naturellement (par exemple, par dessiccation en absence de précipitations dans le milieu naturel de la plante), soit par la main de l'homme par diverses méthodes bien connues de l'homme du métier.

Le terme « *Moricandia* », tel qu'utilisé ici, désigne un genre de plante appartenant à la famille des brassicacées. Le genre inclus une quanrantaine d'espèces différentes, lesquelles sont cataloguées et indexées en ligne, par exemple dans la base de données Species 2000 (Roskov et al., Eds. (2018). Species 2000 & ITIS Catalogue of Life, 30th June 2018. Digital resource at www.catalogueoflife.org/col. Species 2000: Naturalis, Leiden, the Netherlands. ISSN 2405-8858). Une sélection d'espèces représentatives du genre Moricandia comprend, sans y être limité, *Moricandia alypifolia, Moricandia alyssifolia, Moricandia arvensis, Moricandia baetica, Moricandia cinerea, Moricandia clavata, Moricandia crassifolia, Moricandia crenulata, Moricandia divaricata, Moricandia dumosa, Moricandia exacoides, Moricandia foetida, Moricandia foleyi, Moricandia hesperidiflora, Moricandia longirostris, Moricandia meyeri, Moricandia moricandioides, Moricandia nitens, Moricandia pallida, Moricandia papillosa, Moricandia patula, Moricandia perfoliata, Moricandia populifolia, Moricandia ramburii, Moricandia sinaica, Moricandia sonchifolia, Moricandia sonchifolia, Moricandia spinosa, Moricandia suffruticosa, Moricandia teretifolia, Moricandia tortuosa, Moricandia tourneuxii, Moricandia tuberosa* et *Moricandia winkleri*. Tel qu'utilisé ici, le terme « *Moricandia* » couvre cependant l'ensemble des espèces de *Moricandia* connues à ce jour, ainsi que les espèces nouvelles non-découvertes à ce jour.

Le terme « *Moricandia arvensis* », tel qu'utilisé ici, désigne une espèce de plantes appartenant à la famille des brassicacées et distribuée dans la région méditerranéenne, particulièrement dans les régions désertiques du nord de l'Afrique. L'espèce inclus, sans y être limité, les sous-espèces et variétés suivantes : *Moricandia arvensis arvensis, Moricandia arvensis garamantum, Moricandia arvensis nitens, Moricandia arvensis robusta, Moricandia arvensis spinosa* et *Moricandia arvensis suffruticosa*.

Le terme « **obésité** », tel qu'utilisé ici, se réfère à une maladie chronique (telle que reconnue par l'Organisation Mondiale de Santé depuis 1997), définie comme une accumulation anormale ou excessive de graisse corporelle qui peut nuire à la santé. Toujours selon l'Organisation Mondiale de la Santé, la définition de l'obésité repose sur la mesure de l'indice de masse corporelle $\left( IMC = \frac{masse}{taille^2} \right)$, classifiant la maladie en 3 niveaux : la « **surcharge pondérale** » si $25\ kg/m^2 < IMC < 30\ kg/m^2$ ; l'« **obésité** » si $30\ kg/m^2 < IMC < 40\ kg/m^2$ ; et l'« **obésité morbide** » si $40\ kg/m^2 < IMC$.

Le terme « **pharmaceutiquement acceptable** », tel qu'utilisé ici, désigne des molécules et des compositions qui ne produisent pas de réaction indésirable, allergique ou fâcheuse lorsqu'elles sont administrées à un sujet, en particulier à un humain. Sont inclus, sans limitation, tous les solvants, les milieux de dispersion, les enrobages, les agents antibactérien et antifongiques, les agents isotoniques, les agents retardant l'absorption et assimilés. Pour l'administration à l'homme, les préparations doivent respecter les normes de pyrogénicité, de sécurité et de pureté requises par les organismes de réglementation, tel que par exemple la FDA et l'EMA. Un véhicule ou un excipient pharmaceutiquement acceptable peut ainsi se référer à un agent d'encapsulation solide, semi-solide ou liquide ou à une formulation auxiliaire de tout type.

Le terme « **prédiabétique** », tel qu'utilisé ici, se réfère à un sujet souffrant d'une condition médicale dans laquelle sa glycémie à jeun varie d'environ 1 g/L à environ 1,26 g/L de sang (soit d'environ 5,6 mmol/L à environ 7,0 mmol/L de sang), et dans laquelle le risque de développer un diabète de type 2 est augmenté chez ledit sujet. Un sujet prédiabétique répondant aux critères ci-avant porte le diagnostic d'intolérance au glucose.

Le terme « **stéatose hépatique** », tel qu'utilisé ici, se réfère à est une lésion du foie provoquée par une surcharge de vacuoles remplie de triglycérides s'accumulant dans le cytoplasme des hépatocytes. En particulier, la « **stéatose hépatique non-alcoolique** », aussi appelée « **maladie du soda** », est une stéatose sans rapport avec la prise d'alcool.

Le terme « **syndrome métabolique** », tel qu'utilisé ici, se réfère à état pathologique préfigurant des maladies métaboliques plus graves telles que le diabète de type 2. Selon l'Institut National du Coeur, des Poumons et du Sang (NHLBI, Bethesda, MD, États-Unis), on parle de « **syndrome métabolique** » dans les cas où au moins trois des problèmes suivants sont associés chez la même personne : taux d'insuline anormalement élevé, hypercholestérolémie avec un faible taux de cholestérol HDL (< 1,04 mmol/L pour les hommes, < 1,29 mmol/L pour les femmes), hypertension (> 140 mmHg de pression systolique et/ou > 90 mmHg de pression diastolique), excès de poids (notamment obésité abdominale avec un tour de taille > 100 cm pour les hommes, > 88 cm pour les femmes), hyperglycémie (triglycérides sanguins > 1,6 mmol/L et/ou glycémie à jeun > 6,1 mmol/L.

## DESCRIPTION DÉTAILLÉE

La présente invention se rapporte à une composition comprenant au moins un extrait de *Moricandia* pour son utilisation dans la prévention et/ou le traitement d'une maladie métabolique sélectionnée dans le groupe comprenant le diabète, la résistance à l'insuline, l'intolérance au glucose, et l'hyperglycémie.

Dans un mode de réalisation, la composition selon la présente invention comprend, consiste en, ou consiste essentiellement en, au moins un extrait de *Moricandia arvensis.*

Dans un mode de réalisation, la composition selon la présente invention comprend en outre au moins un extrait *d'Astragalus armatus.*

Dans un mode de réalisation, le « au moins un extrait » peut être obtenu par toute méthode d'extraction comprenant une (ou une combinaison de) technique(s) connue(s) de l'homme du métier. Des exemples de techniques d'extraction incluent, mais ne se limitent pas à, la déshydratation, le séchage à l'air, le séchage au micro-ondes, le séchage au four, la lyophilisation, la filtration, le broyage, la macération, la percolation, l'infusion, la décoction, l'extraction au Soxhlet, l'extraction continue à chaud, l'extraction assistée par micro-ondes, l'extraction assistée par ultrasons, l'extraction accélérée par solvant, l'extraction par fluide supercritique, l'extraction aqueuse, l'extraction alcoolique (notamment méthanolique ou éthanolique), la distillation à la vapeur, l'enfleurage, la digestion enzymatique et l'ensemble des techniques apparentées.

Dans un mode de réalisation, le « au moins un extrait » peut être obtenu à partir d'un matériel végétal de départ comprenant ou constitué de la plante entière (*i.e.*, une plante du genre *Moricandia arvensis* ; et/ou une plante du genre *Astragalus armatus*).

Dans un mode de réalisation, le « au moins un extrait » peut être obtenu à partir d'un matériel végétal de départ comprenant ou constitué d'une ou plusieurs parties de la plante. Des exemples de parties de plante qui peuvent être utilisée pour produire un extrait incluent, sans y être limités, les fleurs, les fruits, les graines, le pollen, les racines, les tiges, les feuilles et les écorces.

Dans un mode de réalisation préféré, le « au moins un extrait de *Moricandia arvensis* est un extrait de feuille.

Dans un mode de réalisation préféré, le « au moins un extrait *d'Astragalus armatus* est un extrait de racines.

Dans un mode de réalisation, le matériel végétal est séché après sa récolte.

Dans un mode de réalisation, le matériel végétal est séché à l'air et à l'abri du soleil.

Dans un mode de réalisation, le matériel végétal est séché à l'air et à l'abri du soleil pendant une durée variant d'environ 7 jours à 25 j ours, de préférence d'environ 10 jours à 20 jours, plus préférentiellement pendant une durée d'environ 14 jours.

Dans un mode de réalisation, le matériel végétal est séché à l'air et à l'abri du soleil pendant une durée d'au moins environ 7 jours, de préférence d'au moins environ 7, 8, 9, 10, 11, 12, 13 jours, plus préférentiellement pendant une durée d'au moins environ 14 jours.

Dans un mode de réalisation, le « au moins un extrait » peut être obtenu par une méthode comprenant une étape de décoction.

Dans un mode de réalisation préféré, le « au moins un extrait » de *Moricandia arvensis* peut être obtenu par une méthode comprenant une étape de décoction.

Dans un mode de réalisation, la durée de l'étape de décoction varie d'environ 5 minutes à environ 30 minutes, de préférence d'environ 10 minutes à environ 25 minutes, plus préférentiellement d'environ 15 à environ 20 minutes. Dans un mode de réalisation, la durée de l'étape de décoction est supérieure à environ 5 minutes, de préférence à environ 6, 7, 8, 9, 10, 11, 12, 13 ou 14 minutes, plus préférentiellement à environ 15 minutes.

Dans un mode de réalisation, la température de décoction est supérieure à environ 40°C, de préférence supérieure à environ 45°C, 50°C, 55°C, 60°C, 65°C, 70°C, 75°C, 80°C or 85°C, plus préférentiellement supérieure à environ 90°C. Dans un mode de réalisation préféré, la température de décoction est d'environ 100°C.

Dans un mode de réalisation dans lequel la décoction est réalisée à partir d'un matériel végétal sec, la masse de matière végétale sèche par volume d'eau pour la décoction varie d'environ 1 g/L à environ 200 g/L, de préférence d'environ 50 g/L à 150 g/L, plus préférentiellement la masse de matière végétale sèche par volume d'eau pour la décoction est d'environ 100 g/L. Dans un mode de réalisation dans lequel la décoction est réalisée à partir d'un matériel végétal sec, la masse de matière végétale sèche par volume d'eau pour la décoction est supérieure à environ 1 g/L, de préférence supérieure à environ 10 g/L, plus préférentiellement supérieure à environ 20 g/L, 30 g/L, 40 g/L, 50 g/L, 60 g/L, 70 g/L ou 80 g/L. Dans un mode de réalisation préféré dans lequel la décoction est réalisée à partir d'un matériel végétal sec, la masse de matière végétale sèche par volume d'eau pour la décoction est supérieure à environ 90 g/L.

Dans un mode de réalisation préféré, le « au moins un extrait » de *Moricandia arvensis*, peut être obtenu par une méthode comprenant une étape de décoction de feuilles de *Moricandia arvensis.*

Dans un mode de réalisation, le « au moins un extrait » peut être obtenu par une méthode comprenant une étape de macération.

Dans un mode de réalisation, le « au moins un extrait » peut être obtenu par une méthode comprenant une étape de macération dans un solution de méthanol.

Dans un mode de réalisation préféré, le « au moins un extrait *d'Astragalus armatus* peut être obtenu par une méthode comprenant une étape de macération, de préférence une étape de macération alcoolique (i.*e.*, dans un solvant comprenant une solution alcoolique), plus préférentiellement une étape de macération méthanolique (*i.e.*, dans un solvant comprenant une solution de méthanol).

Dans un mode réalisation, la durée de l'étape de macération varie d'environ 1 jour à environ 30 jours, de préférence d'environ 5 jours à environ 25 jours, plus préférentiellement d'environ 10 j ours à environ 20 j ours. Dans un mode de réalisation, la durée de l'étape de macération est supérieure à 2 jours, de préférence supérieure à 3, 4, 5, 6, 7, 8, 9, 10, 11, 12 ou 13 jours, plus préférentiellement la durée de macération est supérieure à 14 jours. Dans un mode de réalisation préféré, la durée de l'étape de macération est d'environ 15 jours.

Dans un mode réalisation, l'étape de macération est réalisée sans agitation. Dans un mode de réalisation préféré, l'étape de macération est réalisée sous agitation.

Dans un mode de réalisation dans lequel la macération est réalisée à partir d'un matériel végétal sec, la masse de matière végétale sèche par volume de solvant pour la macération varie d'environ 1 g/L à environ 200 g/L, de préférence d'environ 50 g/L à environ 150 g/L, plus préférentiellement la masse de matière végétale sèche par volume de solvant pour la macération est d'environ 100 g/L. Dans un mode de réalisation dans lequel la macération est réalisée à partir d'un matériel végétal sec, la masse de matière végétale sèche par volume de solvant pour la macération est supérieure à 1 g/L, de préférence supérieure à 10 g/L, plus préférentiellement supérieure à 20 g/L, 30 g/L, 40 g/L, 50 g/L, 60 g/L, 70 g/L, 80 g/L ou 90 g/L.

Dans un mode de réalisation préféré, le « au moins un extrait *d'Astragalus armatus* peut être obtenu par une méthode comprenant une étape de macération de racines *d'Astragalus armatus* de préférence une étape de macération alcoolique de racines *d'Astragalus armatus* plus préférentiellement une étape de macération méthanolique de racines *d'Astragalus armatus* .

Dans un mode de réalisation, le « au moins un extrait » peut être obtenu par une méthode comprenant une étape de lyophilisation.

Dans un mode de réalisation, le « au moins un extrait » peut être obtenu par une méthode comprenant une étape de filtration.

Dans un mode de réalisation, le « au moins un extrait » peut être obtenu par une méthode comprenant une étape d'évaporation.

Dans un mode de réalisation préféré, le « au moins un extrait *d'Astragalus armatus* peut être obtenu par une méthode comprenant une étape d'évaporation, préférentiellement une étape d'évaporation sous pression réduite à 40°C.

Dans un mode de réalisation, le « au moins un extrait » est un extrait sec.

Dans un mode de réalisation, le « au moins un extrait » peut être conservé à une température variant d'environ -196°C à environ +40°C, de préférence d'environ -80°C à environ +30°C, de préférence d'environ -20°C à environ +25°C, de préférence à une température d'environ +4°C.

Dans un mode de réalisation, la composition selon la présente invention est une composition pharmaceutique ou un médicament.

La présente invention concerne donc également une composition pharmaceutique comprenant, consistant en ou consistant essentiellement en la composition selon la présente invention et au moins un excipient pharmaceutiquement acceptable.

Des exemples d'excipient pharmaceutiquement acceptables incluent, sans y être limité, des surfactants, des liants, des diluants, des lubrifiants, des conservateurs, des stabilisateurs, des antioxydants, des suspensions, des systèmes d'administration, échangeurs d'ions, de l'alumine, du stéarate d'aluminium, de la lécithine, des protéines du sérum, telles que sérumalbumine humaine, des substances tampons telles que les phosphates, la glycine, l'acide sorbique, le sorbate de potassium, les mélanges de glycérides partiels d'acides gras végétaux saturés, l'eau, des sels ou des électrolytes, tels que sulfate de protamine, hydrogénophosphate de sodium, hydrogénophosphate de potassium, le chlorure de sodium, les sels de zinc, de la silice, de la silice colloïdale, du trisilicate de magnésium, du polyvinylpyrrolidone, des substances à base de cellulose (par exemple carboxyméthylcellulose sodique), du polyéthylène glycol, des polyacrylates, des cires et de la lanoline.

La présente invention concerne donc également un médicament comprenant, consistant en ou consistant essentiellement en la composition selon la présente invention.

Dans un mode de réalisation, la composition selon la présente invention est une composition nutraceutique ou alimentaire. Une telle composition est également connue sous le terme d' « **alicament** », *i.e.*, un aliment dont la formulation implique un effet actif sur la santé et/ou le bien-être du sujet le consommant.

La présente invention concerne donc également une composition nutraceutique ou alimentaire comprenant, consistant en ou consistant essentiellement en la composition selon l'invention.

Dans un mode de réalisation, la présente invention se rapporte à la composition, la composition pharmaceutique, la composition nutraceutique ou le médicament tels que décrits ci-dessus, pour leur utilisation dans la prévention et/ou le traitement des maladies métaboliques sélectionnées dans le groupe comprenant le diabète, la résistance à l'insuline, l'intolérance au glucose, et l'hyperglycémie.

Des exemples de maladies métaboliques incluent, sans y être limité, le diabète (par exemple, le diabète de type 2 ou le diabète de type 1), la résistance à l'insuline, l'intolérance au glucose, et l'hyperglycémie.

Des exemples de maladies métaboliques liée à une intolérance au glucose et/ou à une résistance à l'insuline incluent, sans y être limité, le diabète (par exemple, le diabète de type 2 ou le diabète de type 1), le syndrome métabolique, les maladies liées à la résistance à ou une déficience en insuline, l'intolérance au glucose, l'hyperglycémie, l'obésité, la dyslipidémie, l'hypercholestérolémie, l'hypertriglycéridémie et le stress oxydatif.

Selon un mode de réalisation préféré, la maladie métabolique, de préférence la maladie métabolique liée à une intolérance au glucose et/ou à une résistance à l'insuline, est sélectionnée parmi le diabète de type 2, la résistance à l'insuline, l'intolérance au glucose et l'hyperglycémie.

Selon un mode réalisation préféré, la maladie métabolique, de préférence la maladie métabolique liée à une intolérance au glucose et/ou à une résistance à l'insuline, est le diabète. Selon un mode réalisation préféré, la maladie métabolique, de préférence la maladie métabolique liée à une intolérance au glucose et/ou à une résistance à l'insuline, est le diabète de type 2.

Dans un mode de réalisation, la présente invention se rapporte à la composition, la composition pharmaceutique, la composition nutraceutique ou le médicament tels que décrits ci-dessus, pour leur utilisation dans une méthode d'augmentation de la tolérance au glucose chez un sujet. Dans un mode de réalisation, la présente invention se rapporte à une méthode d'augmentation de la tolérance au glucose chez un sujet, comprenant l'administration de la composition, la composition pharmaceutique, la composition nutraceutique ou le médicament tels que décrits ci-dessus.

Les techniques permettant d'évaluer la tolérance au glucose chez un sujet sont bien connues de la personne du métier. Par exemple, la tolérance au glucose peut être évaluée par un test de tolérance au glucose, tel qu'il sera décrit dans la section Exemples ci-dessous.

Dans un mode de réalisation, la présente invention se rapporte à la composition, ou la composition nutraceutique tels que décrits ci-dessus, pour leur utilisation dans la réduction de l'augmentation de la proportion de masse grasse chez un sujet. Dans un mode de réalisation, la présente invention se rapporte à une méthode de réduction de l'augmentation de la proportion de masse grasse chez un sujet, comprenant l'administration de la composition, ou la composition nutraceutique tels que décrits ci-dessus.

Dans un mode de réalisation, la présente invention se rapporte à la composition, ou la composition nutraceutique tels que décrits ci-dessus, pour leur utilisation dans la réduction de la diminution de la proportion de masse maigre chez un sujet. Dans un mode de réalisation, la présente invention se rapporte à une méthode de réduction de la diminution de la proportion de masse maigre chez un sujet, comprenant l'administration de la composition, ou la composition nutraceutique tels que décrits ci-dessus.

Les techniques permettant de mesurer la proportion de masse grasse et/ou de masse maigre chez un sujet sont connues de la personne du métier. Par exemple, la composition corporelle peut être mesurée par l'imagerie par résonance magnétique, tel qu'il sera décrit dans la section Exemples ci-dessous.

Dans un mode de réalisation, la présente invention se rapporte à la composition, la composition pharmaceutique, la composition nutraceutique ou le médicament tels que décrits ci-dessus, pour leur utilisation dans l'augmentation de la sécrétion d'insuline chez un sujet. Dans un mode de réalisation, la présente invention se rapporte à une méthode d'augmentation de la sécrétion d'insuline chez un sujet, comprenant l'administration de la composition, la composition pharmaceutique, la composition nutraceutique ou le médicament tels que décrits ci-dessus.

Les techniques permettant de mesurer la sécrétion d'insuline chez un sujet sont bien connues de la personne du métier. Par exemple, l'insulinémie (basale ou après hyperglycémie induite par voie orale) peut être mesurée pour déterminer le taux d'insuline sécrété chez le sujet, tel qu'il sera décrit dans la section Exemples ci-dessous.

Dans un mode de réalisation, la présente invention se rapporte à la composition, la composition pharmaceutique, la composition nutraceutique ou le médicament tels que décrits ci-dessus, pour leur utilisation dans la normalisation de la glycémie chez un sujet. Dans un mode de réalisation, la présente invention se rapporte à une méthode de normalisation de la glycémie chez un sujet, comprenant l'administration de la composition, la composition pharmaceutique, la composition nutraceutique ou le médicament tels que décrits ci-dessus.

On entend par « **normalisation de la glycémie** », le retour de la concentration de glucose mesurée dans le sang du sujet à un niveau variant d'environ 0,5 g/L à environ 1,26 g/L, de préférence d'environ 0,7 g/L à environ 1,10 g/L, de préférence d'environ 0.7 g/L à environ 1,0 g/L.

Dans un mode de réalisation, la présente invention se rapporte à la composition, ou la composition nutraceutique tels que décrits ci-dessus, pour leur utilisation dans la prévention de la prise de poids chez un sujet. Dans un mode de réalisation, la présente invention se rapporte à une méthode de prévention de la prise de poids chez un sujet, comprenant l'administration de la composition, ou la composition nutraceutique tels que décrits ci-dessus.

Dans un mode de réalisation, la présente invention se rapporte à la composition, ou la composition nutraceutique tels que décrits ci-dessus, pour leur utilisation dans la prévention de la prise de masse grasse chez un sujet. Dans un mode de réalisation, la présente invention se rapporte à une méthode de prévention de la prise de masse grasse chez un sujet, comprenant l'administration de la composition, ou la composition nutraceutique tels que décrits ci-dessus.

Dans un mode de réalisation, la présente invention se rapporte à la composition, ou la composition nutraceutique tels que décrits ci-dessus, pour leur utilisation dans le contrôle de la prise de poids chez un sujet. Dans un mode de réalisation, la présente invention se rapporte à une méthode de contrôle de la prise de poids chez un sujet, comprenant l'administration de la composition, ou la composition nutraceutique tels que décrits ci-dessus.

Dans un mode de réalisation, la présente invention se rapporte à la composition, ou la composition nutraceutique tels que décrits ci-dessus, pour leur utilisation dans le contrôle de la prise de masse grasse chez un sujet. Dans un mode de réalisation, la présente invention se rapporte à une méthode de contrôle de la prise de masse grasse chez un sujet, comprenant l'administration de la composition, ou la composition nutraceutique tels que décrits ci-dessus.

Dans un mode de réalisation, la présente invention se rapporte à la composition, ou la composition nutraceutique tels que décrits ci-dessus, pour leur utilisation dans la stimulation de la perte de poids chez un sujet. Dans un mode de réalisation, la présente invention se rapporte à une méthode de stimulation de la perte de poids chez un sujet, comprenant l'administration de la composition, ou la composition nutraceutique tels que décrits ci-dessus.

Dans un mode de réalisation, la présente invention se rapporte à la composition, ou la composition nutraceutique tels que décrits ci-dessus, pour leur utilisation dans la stimulation de la perte de masse grasse chez un sujet. Dans un mode de réalisation, la présente invention se rapporte à une méthode de stimulation de la perte de masse grasse chez un sujet, comprenant l'administration de la composition, ou la composition nutraceutique tels que décrits ci-dessus.

Dans un mode de réalisation, la présente invention se rapporte à une méthode cosmétique (ou non-thérapeutique) comprenant l'administration de la composition ou la composition nutraceutique selon l'invention.

Dans un mode de réalisation, la méthode cosmétique ou non-thérapeutique selon la présente invention est destinée à prévenir la prise de poids chez un sujet. Dans un mode de réalisation, la méthode cosmétique ou non-thérapeutique selon la présente invention est destinée à prévenir la prise de masse grasse chez un sujet.

Dans un mode de réalisation, la méthode cosmétique ou non-thérapeutique selon la présente invention est destinée à contrôler la prise de poids chez un sujet. Dans un mode de réalisation, la méthode cosmétique ou non-thérapeutique selon la présente invention est destinée à contrôler la prise de masse grasse chez un sujet.

Dans un mode de réalisation, la méthode cosmétique ou non-thérapeutique selon la présente invention est destinée à stimuler la perte de poids chez un sujet. Dans un mode de réalisation, la méthode cosmétique ou non-thérapeutique selon la présente invention est destinée à stimuler la perte de masse grasse chez un sujet.

Dans un mode de réalisation préféré, les méthodes cosmétiques (ou non-thérapeutiques) selon la présente invention sont destinées à des sujets substantiellement sains. On entend par « **substantiellement sain** » un sujet qui n'est pas affecté par et/ou n'a pas été diagnostiqué avec une maladie métabolique, de préférence avec une maladie métabolique liée à une intolérance au glucose et/ou à une résistance à l'insuline, telle que définie ci-dessus.

Dans un mode de réalisation, la composition, la composition pharmaceutique, la composition nutraceutique ou le médicament selon l'invention est administré(e) de manière systémique ou locale.

Dans un mode de réalisation, la composition, la composition pharmaceutique, la composition nutraceutique ou le médicament selon l'invention est administré(e) par voie orale, par voie buccale, par injection, par voie percutanée, par voie parentérale, par voie intrapéritonéale, par voie rectale, par voie vaginale, par voie topique transdermique ou transmucosale, par voie nasale, par inhalation ou par l'utilisation d'un réservoir implanté.

Dans un mode de réalisation, la composition, la composition pharmaceutique, la composition nutraceutique ou le médicament selon l'invention est administré(e) par voie orale.

Dans ce contexte, différentes formes galéniques sont possibles, comme notamment des formes solides, semi-solides, souples, liquides, vaporisées ou pressurisées, vaporisations et diffusions ultrasoniques.

Des exemples de formes solides adaptées à une administration par voie orale incluent, sans y être limité, les pilules, pastilles, granules, granulés, poudres, gommes ou pâtes à mâcher, capsules à enveloppe molle, gélules, cachets, comprimés enrobés ou non, comprimés effervescent, comprimés à libération modifiée, comprimés orodispersibles ou gastrorésistants, dragées et autres formes solides adaptées à la préparation de solution ou suspension avant l'administration par voie orale telles que des poudres orales, poudres lyophilisées soluble dans un liquide chaud ou froid.

Des exemples de formes liquides adaptées à une administration par voie orale incluent, sans y être limités, les sirops, émulsions, solutions, suspensions, infusions, tisanes, teintures-mère, teintures officinales, macérats, macérats huileux, huiles végétales, macérats hydroalcooliques glycérinés, décoctions, nébuliseurs et aux sprays.

Des exemples de formes galéniques pressurisées ou pneumatiques incluent, sans y être limités, les nébuliseurs, inhalateurs et diffuseurs soniques.

Dans un mode de réalisation, la composition, la composition pharmaceutique, la composition nutraceutique ou le médicament selon l'invention, plus préférentiellement la composition nutraceutique, peut également se présenter sous forme d'un complément ou additif alimentaire. Des exemples de tels compléments ou additifs incluent, sans y être limités, les compléments solides (tels que les broyats et brisures de plantes séchées ou fraiches ou d'une partie de plante séchée ou fraiche, les poudres à diluer ou à mélanger à l'alimentation, les barres alimentaires, etc.) et les compléments liquides (tels que les boissons, sirops, émulsions, solutions, infusions, tisanes, teintures-mère, macérats, macérats huileux, macérats hydroalcooliques glycérinés et autres décoctions). Le terme boisson tel qu'utilisé ici inclut, sans y être limité, les boissons alcoolisées, boissons non alcoolisées, thés, infusions, tisanes, décoctions, boissons énergétiques, jus de fruits, limonades, sodas, produits laitiers, soupes. Dans le cas des compléments liquides plus spécifiquement, ceux-ci peuvent se présenter sous diverses formes telles que des compléments prêts à consommer ou des concentrés à diluer.

Dans un mode de réalisation, la composition, la composition pharmaceutique, la composition nutraceutique ou le médicament selon l'invention est administré(e) par injection, préférentiellement par injection systémique.

Dans un mode de réalisation, la composition, la composition pharmaceutique, la composition nutraceutique ou le médicament selon l'invention est administré(e) par injection sous-cutanée.

Dans un mode de réalisation, la composition, la composition pharmaceutique, la composition nutraceutique ou le médicament selon l'invention est administré(e) par injection intrapéritonéale.

Des exemples de formulation adaptée à l'administration par injection incluent, mais ne se limitent pas, aux solutions, suspensions, formes solides pour la préparation de solution ou suspensions injectables.

Dans un mode de réalisation, la composition, la composition pharmaceutique, la composition nutraceutique ou le médicament selon l'invention est administré(e) à une dose adaptée à chaque sujet et déterminée par une personne qualifiée.

Il est ainsi précisé que la dose quotidienne de la composition, de la composition pharmaceutique, la composition nutraceutique ou du médicament selon l'invention sera décidée par le médecin traitant dans le cadre d'une pratique médicale réfléchie. Cette dose telle que définie pour un sujet en particulier peut varier en fonction de facteurs divers incluant notamment : la maladie et sa sévérité ; la composition utilisée ; l'âge, le poids, la santé, le sexe et le régime alimentaire du sujet; l'heure d'administration; la voie d'administration ; la durée du traitement ; d'autre médicaments utilisés en parallèle ou en combinaison avec la composition, la composition pharmaceutique, la composition nutraceutique ou la médicament selon l'invention et l'ensemble dans facteurs assimilés connus dans le cadre de la pratique médicale. Par exemple, il est possible de commencer un traitement à une dose inférieure à celle requise pour obtenir l'effet thérapeutique désiré et ensuite augmenter progressivement le dosage jusqu'à l'obtention de l'effet thérapeutique désiré. À l'opposé, il peut être judicieux de commencer le traitement par un bolus (ou dose d'attaque) de façon à atteindre rapidement la concentration plasmatique désirée et ensuite compenser l'élimination par l'administration de doses de maintenance.

Dans un mode de réalisation, la composition, la composition pharmaceutique, la composition nutraceutique ou le médicament selon l'invention est administré(e) au moins une fois par jour, au moins deux fois par jour, au moins trois fois par jour.

Dans un mode de réalisation, la composition, la composition pharmaceutique, la composition nutraceutique ou le médicament selon l'invention est administré(e) tous les deux, trois, quatre, cinq, six jours.

Dans un mode de réalisation, la composition, la composition pharmaceutique, la composition nutraceutique ou le médicament est administré(e) deux fois par semaine, une fois par semaine, toutes les deux semaines, toutes les trois semaines, une fois par mois.

Dans un mode de réalisation, la composition, la composition pharmaceutique, la composition nutraceutique ou le médicament selon l'invention est administré(e) chaque mois, tous les deux mois, tous les trois mois, tous les quatre mois, tous les cinq mois, tous les six mois, une fois par an.

Dans un mode de réalisation, la composition, la composition pharmaceutique, la composition nutraceutique ou le médicament selon l'invention est administré(e) durant un période d'environ un jour, deux jours, trois j ours, quatre j ours, cinq j ours, six jours, une semaine, deux semaines, trois semaines, un mois, deux mois, trois mois, six mois, un an, ou durant une période plus longue, par exemple plusieurs années ou durant l'ensemble de la vie restante du sujet.

Dans un mode de réalisation, la composition, la composition pharmaceutique, la composition nutraceutique ou le médicament selon l'invention est administré(e) jusqu'au traitement ou jusqu'à l'amélioration des symptômes associés à la maladie métabolique, de préférence à la maladie métabolique liée à une intolérance au glucose et/ou à une résistance à l'insuline.

Dans un mode de réalisation, la composition, la composition pharmaceutique, la composition nutraceutique ou le médicament selon l'invention est administré(e) dans le cadre d'un traitement chronique. Dans un mode de réalisation, la composition, la composition pharmaceutique, la composition nutraceutique ou le médicament selon l'invention est administré(e) dans le cadre d'un traitement ponctuel.

Dans un mode de réalisation alternatif, la composition, la composition pharmaceutique, la composition nutraceutique ou le médicament selon l'invention est administré(e) *pro re nata*, *i.e.*, au besoin. Les termes « **administré au besoin** » et « ***pro re nata*** », tels qu'utilisés ici, se réfèrent à ces compositions, compositions pharmaceutiques, compositions nutraceutiques ou médicaments pour lesquels le dosage et de la fréquence de prise sont laissés à la discrétion du sujet, par opposition aux compositions qui doivent être administrées selon un dosage et une fréquence fixe.

Dans un mode de réalisation, la dose quotidienne de *Moricandia* dans la composition, la composition pharmaceutique, la composition nutraceutique ou le médicament selon l'invention qui peut ou doit être administrée au sujet varie d'environ 10 mg/kg à environ 500 mg/kg, d'environ 50 mg/kg à environ 450 mg/kg, d'environ 100 mg/kg à environ 400 mg/kg, d'environ 150 mg/kg à environ 350 mg/kg, d'environ 175 mg/kg à environ 325 mg/kg, d'environ 200 mg/kg à environ 300 mg/kg, d'environ 225 mg/kg à environ 275 mg/kg, d'environ 240 mg/kg à environ 260 mg/kg. Dans un mode de réalisation, la dose quotidienne de la composition, la composition pharmaceutique, la composition nutraceutique ou du médicament selon l'invention qui peut ou doit être administrée au sujet est d'environ 250 mg/kg.

Dans un mode de réalisation, la dose humaine équivalente quotidienne de *Moricandia* dans la composition, la composition pharmaceutique, la composition nutraceutique ou le médicament selon l'invention qui peut ou doit être administrée au sujet varie d'environ 1 mg/kg à environ 80 mg/kg, d'environ 7,5 mg/kg à environ 72,5 mg/kg, d'environ 15 mg/kg à environ 65 mg/kg, d'environ 22,5 mg/kg à environ 57,5 mg/kg, d'environ 27,5 mg/kg à environ 52,5 mg/kg, d'environ 32,5 mg/kg à environ 47,5 mg/kg, d'environ 37,5 mg/kg à environ 42,5 mg/kg, d'environ 39 mg/kg à environ 41 mg/kg. Dans un mode de réalisation, la dose quotidienne de la composition, la composition pharmaceutique, la composition nutraceutique ou du médicament selon l'invention qui peut ou doit être administrée au sujet est d'environ 40 mg/kg.

Dans un mode de réalisation, la dose quotidienne d'*Astragalus* dans la composition, la composition pharmaceutique, la composition nutraceutique ou le médicament selon l'invention qui peut ou doit être administrée au sujet varie d'environ 10 mg/kg à environ 500 mg/kg, d'environ 50 mg/kg à environ 450 mg/kg, d'environ 100 mg/kg à environ 400 mg/kg, d'environ 150 mg/kg à environ 350 mg/kg, d'environ 175 mg/kg à environ 325 mg/kg, d'environ 200 mg/kg à environ 300 mg/kg, d'environ 225 mg/kg à environ 275 mg/kg, d'environ 240 mg/kg à environ 260 mg/kg. Dans un mode de réalisation, la dose quotidienne de la composition, la composition pharmaceutique, la composition nutraceutique ou du médicament selon l'invention qui peut ou doit être administrée au sujet est d'environ 250 mg/kg.

Dans un mode de réalisation, la dose humaine équivalente quotidienne d'*Astragalus* dans la composition, la composition pharmaceutique, la composition nutraceutique ou le médicament selon l'invention qui peut ou doit être administrée au sujet varie d'environ 1 mg/kg à environ 80 mg/kg, d'environ 7,5 mg/kg à environ 72,5 mg/kg, d'environ 15 mg/kg à environ 65 mg/kg, d'environ 22,5 mg/kg à environ 57,5 mg/kg, d'environ 27,5 mg/kg à environ 52,5 mg/kg, d'environ 32,5 mg/kg à environ 47,5 mg/kg, d'environ 37,5 mg/kg à environ 42,5 mg/kg, d'environ 39 mg/kg à environ 41 mg/kg. Dans un mode de réalisation, la dose quotidienne de la composition, la composition pharmaceutique, la composition nutraceutique ou du médicament selon l'invention qui peut ou doit être administrée au sujet est d'environ 40 mg/kg.

Dans un mode de réalisation, les doses quotidiennes présentées ci-dessus correspondent aux doses de matière végétale de *Moricandia* et/ou d'*Astragalus*, préférentiellement aux doses de matière végétale sèche de *Moricandia* et/ou d'*Astragalus.*

Dans un mode de réalisation préféré, les doses quotidiennes présentées ci-dessus correspondent aux doses d'extrait de *Moricandia* et/ou d'*Astragalus* obtenus après extraction comme présenté plus haut ; de manière préférée par déshydratation, séchage à l'air, séchage au micro-ondes, séchage au four, lyophilisation, filtration, broyage, macération, percolation, infusion, décoction, extraction au Soxhlet, extraction continue à chaud, extraction assistée par micro-ondes, extraction assistée par ultrasons, extraction accélérée par solvant, extraction par fluide supercritique, extraction aqueuse, extraction alcoolique, distillation à la vapeur, enfleurage, digestion enzymatique ou une combinaison de deux ou plus de ces techniques ; de manière plus préférée par décoction ou macération ; de manière encore plus préférée, par lyophilisation après décoction ou macération.

Dans un mode réalisation, le sujet est humain.

Dans un mode de réalisation, le sujet a été diagnostiqué, ou est à risque d'être diagnostiqué, avec une maladie métabolique, de préférence avec une maladie métabolique liée à une intolérance au glucose et/ou à une résistance à l'insuline.

Dans un mode de réalisation, le sujet est prédiabétique. Dans un mode de réalisation, le sujet est diabétique.

On entend par « **prédiabétique** », un état caractérisé par une glycémie plus élevée que la normale, mais pas suffisamment pour établir le diagnostic du diabète. Typiquement, un sujet est diagnostiqué comme étant « **prédiabétique** » lorsque sa glycémie à jeun se situe entre 6,1 et 6,9 mmol/L ; ou son taux d'hémoglobine glyquée dans le sang se situe entre 6,0 et 6,4% ; et/ou sa glycémie mesurée deux heures après ingestion de 75 g de glucose se situe entre 7,8 et 11,0 mmol/L.

Dans un mode de réalisation, le sujet présente un risque élevé de développer un diabète de type 2. Des exemples de facteurs de risques associés au diabète de type 2 incluent, sans y être limité, la condition prédiabétique, l'hypertension artérielle, le genre masculin, l'âge supérieur à 40 ans, le tour de taille élevé, la faible activité physique, l'alimentation sucrée et/ou grasse et les prédispositions liées à l'hérédité (génétique et environnementale).

## BRÈVE DESCRIPTION DES FIGURES

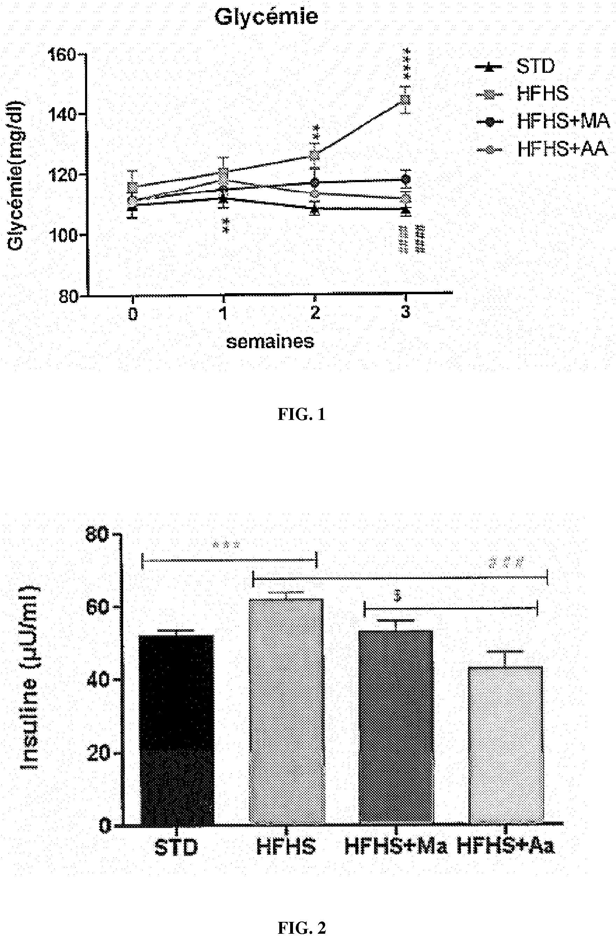

La **Figure 1** est un graphique montrant l'évolution de la glycémie au cours du protocole d'étude dans les différents groupes de rats. La glycémie a été mesurée au début du protocole et à la fin de chacune des trois premières semaines. La légende du graphique se lit comme suit :

- STD : rats suivant un régime alimentaire standard, *i.e.*, groupe de référence ;
- HFHS (« High Fat High Sucrose ») : rats suivant un régime alimentaire riche en sucre et matières grasses, diabétiques à l'issue des 3 semaines ;
- HFHS-MA (« High Fat High Sucrose ») : rats suivant un régime alimentaire riche en sucre et matières grasses et traités chaque matin avec 250 mg/kg par gavage avec un extrait de *Moricandia arvensis* dès le début du régime HFHS ;
- HFHS-AA (« High Fat High Sucrose ») : rats suivant un régime alimentaire riche en sucre et matières grasses et traités chaque matin dès le début du régime HFHS avec 250 mg/kg par gavage avec un extrait d'*Astragalus armatus.*

   ** $p < 0,01$ et **** $p < 0,0001$ : STD vs. HFHS
   #### $p < 0,001$ : HFHS vs. HFHS+MA et HFHS vs. HFHS+AA
   Test ANOVA 2 voies.

La **Figure 2** est un histogramme montrant l'insulinémie basale après 3 semaines du protocole d'étude dans les différents groupes de rats tels que définis dans la légende de la **Figure 1.**

   *** $p < 0,001$
   ### $p < 0,001$
   $ $p < 0,05$
   Test ANOVA 2 voies.

La **Figure 3** montre les résultats du test de sensibilité à l'insuline réalisé à la fin des 3 semaines du protocole d'étude dans les différents groupes de rats tels que définis dans la légende de la **Figure 1.**

   **(A)** Graphique illustrant l'évolution de la glycémie au cours du test. Après 45 min suivant l'injection d'insuline, alors que la glycémie ne baisse plus dans le groupe HFHS, les groupes HFHS+MA et AA présentent une glycémie qui n'est pas significativement différente des témoins et très significativement diminuée comparée au groupe HFHS non traité.

   ** $p < 0,01$ et **** $p < 0,0001$ : HFHS vs. HFHS-MA
   ## $p < 0,01$ et #### $p < 0,0001$ : HFHS vs. STD
   ### $p < 0,001$ et #### $p < 0,0001$ : HFHS vs. HFHS-AA
   Test ANOVA 2 voies.

   **(B)** Histogramme illustrant l'aire sous les courbes du graphique **(A)**. AUC : aire sous la courbe (en anglais, *Area Under Curve*).

   ** $p < 0,01$ : HFHS vs. STD
   # $p < 0,05$ : HFHS vs. HFHS+MA et HFHS+AA vs. HFHS
   Test ANOVA 2 voies.

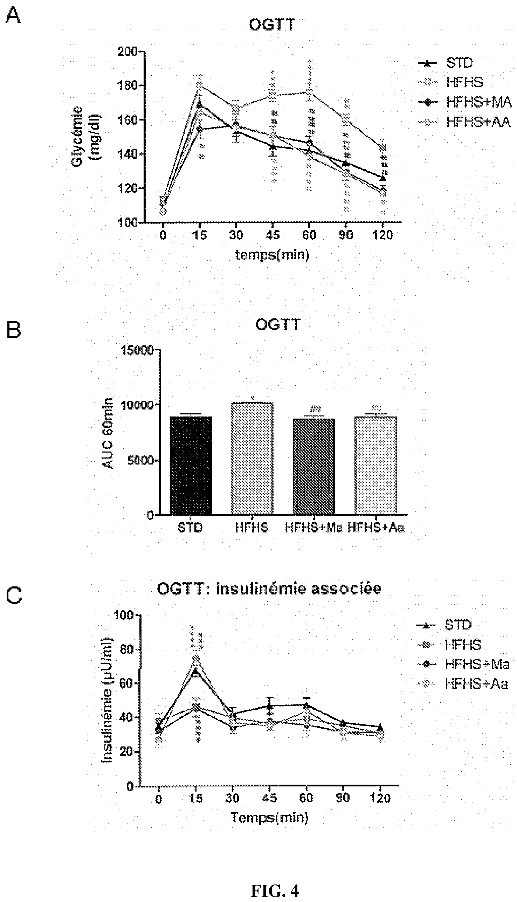

La **Figure 4** montre les résultats du test de tolérance au glucose (2 g/kg par voie orale) réalisé à 3 semaines du protocole d'étude dans les différents groupes de rats tels que définis dans la légende de la **Figure 1.**

   **(A)** Graphique illustrant l'évolution de la glycémie au cours du test. Les animaux traités par AA et MA ont une meilleure tolérance au glucose comparée aux animaux non traités.

   ** $p < 0,01$ et *** $p < 0,001$ et **** $p < 0,0001$ : HFHS vs. STD
   ## $p < 0,01$ et ### $p < 0,001$ et #### $p < 0.0001$ : HFHS-MA vs. HFHS
   ## $p < 0,01$ et #### $p < 0,0001$ : HFHS vs. HFHS-AA
   Test ANOVA 2 voies.

   **(B)** Histogramme illustrant l'aire intégrée sous la courbe (glycémie en fonction du temps dans l'intervalle 0-60 minutes lors du test). L'intolérance au glucose observée chez les rats HFHS n'est pas présente quand le régime HFHS a été associé au traitement MA ou AA.

* p<0,05 : HFHS vs. STD
## p<0,01 : HFHS-MA vs. HFHS et HFHS vs. HFHS-AA
Test ANOVA 2 voies.

**(C)** Graphique illustrant l'évolution de l'insulinémie au cours du test OGTT. La sécrétion d'insuline en réponse au glucose est altérée chez les rats HFHS. La sécrétion d'insuline en réponse au glucose reste altérée chez les rats HFHS traités avec MA. Chez les rats HFHS traités avec AA la sécrétion d'insuline en réponse au glucose est équivalente aux témoins.

* p<0,05 : HFHS vs. STD
## p<0,01 : HFHS-MA vs. HFHS et HFHS vs. HFHS-AA
Test ANOVA 2 voies.

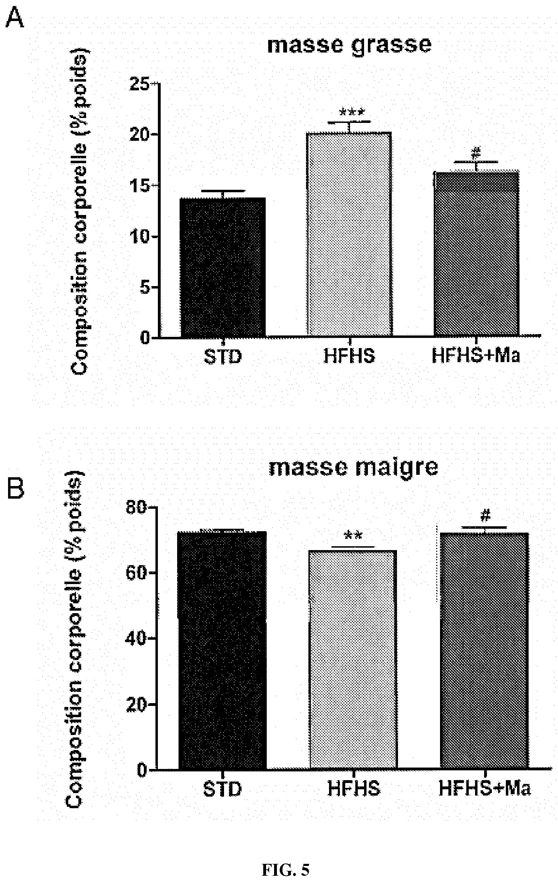

La **Figure 5** montre les résultats de la mesure de la composition corporelle après 3 semaines (1 à 3 jours suivant les 3 semaines du protocole d'étude) dans les différents groupes de rats.

- STD : rats suivant un régime alimentaire standard ;
- HFHS (« High Fat High Sucrose ») : rats suivant un régime alimentaire riche en sucre et matières grasses ;
- HFHS-MA (« High Fat High Sucrose ») : rats suivant un régime alimentaire riche en sucre et matières grasses et traités chaque matin avec 250 mg/kg par gavage avec un extrait de *Moricandia arvensis*.

**(A)** Histogramme illustrant la proportion de la masse grasse en % du poids total ; les % d'eau corporelle ne sont pas représentés. Le traitement MA permet d'empêcher la prise de masse grasse pendant le régime HFHS.

*** p<0,001 : HFHS vs. STD
# p<0,05 : HFHS-MA vs. HFHS
Test ANOVA 2 voies.

**(B)** Histogramme illustrant la proportion de la masse maigre. Le traitement MA permet d'empêcher la baisse de masse maigre pendant le régime HFHS.

** p<0,01 : HFHS vs. STD
# p<0,05 : HFHS-MA vs. HFHS
Test ANOVA 2 voies.

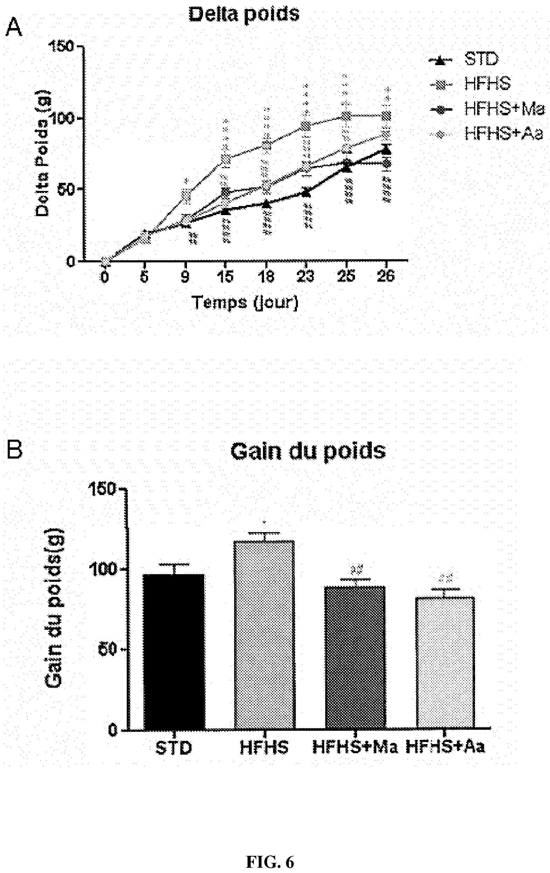

La **Figure 6** montrant l'évolution des différences de poids (delta poids) au cours du protocole d'étude dans les différents groupes de rats tels que définis dans la légende de la **Figure 1.**

**(A)** Graphique illustrant l'évolution du poids au cours du protocole d'étude. Les animaux sous régime HFHS présentent une prise de poids supérieure à celle des rats témoins STD. Le traitement avec MA ou avec AA prévient de la prise de poids chez les animaux HFHS.

* p< 0,05 et ** p<0,01 et **** p<0,0001 : STD *vs.* HFHS
## p<0.01 et #### p<0,001 : HFHS *vs.* HFHS+MA
## p< 0,01 et ### p< 0,001 : HFHS *vs.* HFHS+AA
Test ANOVA 2 voies.

**(B)** Histogramme illustrant le gain de poids au cours du protocole d'étude. Les animaux HFHS traités avec MA ou AA présentent une prise de poids identique aux rats témoins STD et significativement plus faible que les rats HFHS sans traitement.

* p<0,05 et ** p<0,01 : STD *vs.* HFHS
## p<0.01 : HFHS *vs.* HFHS+MA et HFHS *vs.* HFHS+AA
Test ANOVA 2 voies.

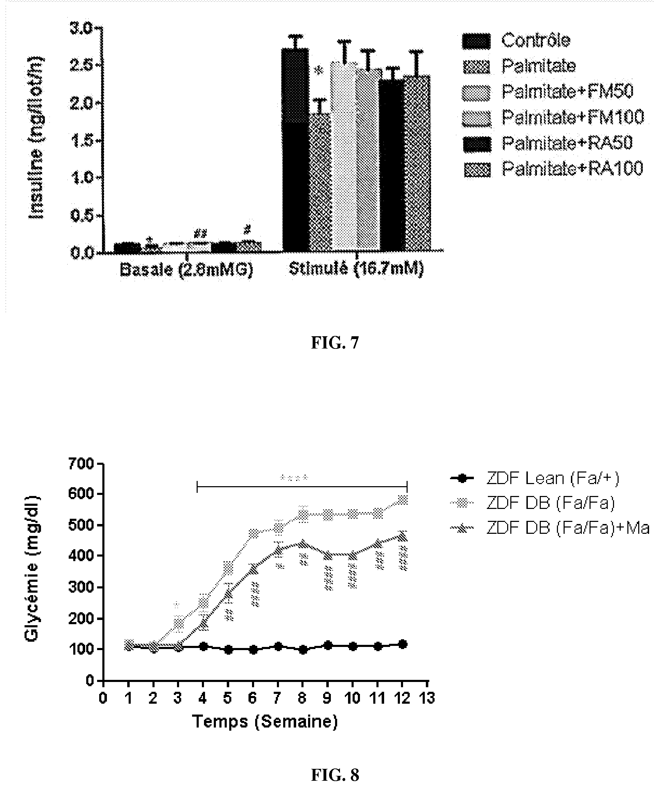

La **Figure 7** est un histogramme montrant la capacité de sécrétion d'insuline d'îlots de Langerhans primaires de souris C57Bl6 en culture pendant 72 heures dans les conditions suivantes :

- contrôle : milieu de culture seul ;
- palmitate : milieu de culture + 0,5 mM palmitate (conditions lipotoxiques mimant l'environnement d'un DT2) ;
- palmitate + FM50 (*M.arvensis* 50 mg) : milieu de culture + 0,5 mM palmitate + 50 $\mu$g/mL d'extrait de feuilles de *Moricandia arvensis* ;
- palmitate + FM100 (*M.arvensis* 100 mg) : milieu de culture + 0,5 mM palmitate + 100 $\mu$g/mL d'extrait de feuilles *Moricandia arvensis* ;
- palmitate + RA50 (*A.armatus* 50 mg) : milieu de culture + 0,5 mM palmitate + 50 $\mu$g/mL d'extrait de racine *d'Astragalus armatus* ;
- palmitate + RA100 *(A.armatus* 100 mg): milieu de culture + 0,5 mM palmitate + 100 $\mu$g/mL d'extrait de racine *d'Astragalus armatus.*

La sécrétion d'insuline a été mesurée *in vitro* soit en conditions basales (concentration de glucose non-stimulante : 2,8 mM), soit après stimulation (glucose 16,7 mM) à la fin des conditions de culture prédéfinies. Le traitement avec les extraits *Moricandia arvensis* ou *Astragalus armatus* en condition « palmitate » restaure une sécrétion d'insuline similaire à celle des îlots en condition normale.

* $p < 0,05$ : Contrôle vs. Palmitate
## $p < 0,01$ : Palmitate vs. P+FM100
# $p < 0,05$ : Palmitate *vs*. P+RA100

La **Figure 8** est un graphique montrant l'évolution de la glycémie au cours du protocole d'étude dans les différents groupes de rats ZDF. Le suivi est réalisé à la fin de chaque semaine pendant 12 semaines.

- ZDF Lean (*fa*/+) : contrôle hétérozygote pour la mutation récessive ou homozygotes pour l'allèle sauvage du récepteur à la leptine, *i.e.*, groupe de référence.
- ZDF DB (*fa*/*fa*) : homozygotes pour la mutation du récepteur à la leptine. Les animaux sont obèses, insulino-résistants puis diabétiques de façon irréversible.
- ZDF DB (*fa*/*fa*) + MA : homozygotes pour la mutation du récepteur à la leptine traité avec 250 mg/kg par gavage avec un extrait de *Moricandia arvensis* préparé comme décrit dans la **Figure 1** ci-dessus.

* $p < 0,05$ et **** $p < 0,001$ : ZDF DB *vs*. ZDF lean
# $p < 0,05$ et ## $p < 0.01$ et ### $p < 0,001$ et #### $p < 0,0001$ : ZDF DB+ MA vs. ZDF DB
Test ANOVA deux voies.

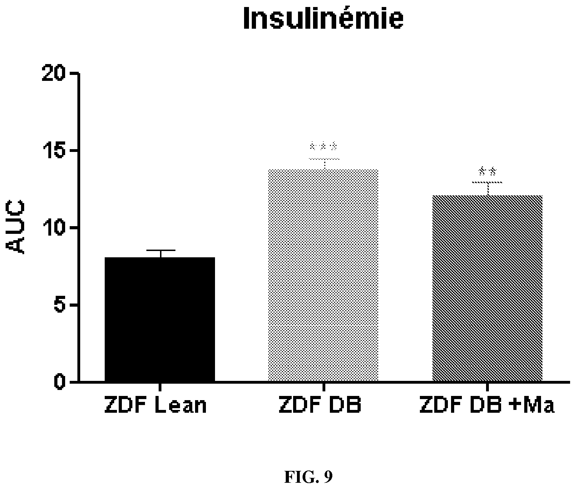

La **Figure 9** est un histogramme montrant l'évolution de l'insulinémie au cours du temps (intégration de l'aire sous la courbe) pendant les 8 premières semaines du protocole d'étude dans les différents groupes de rats tels que définis dans la légende de la **Figure 8.**

** $p < 0,01$ et *** $p < 0,001$ : ZDF DB *vs*. ZDF lean
Test ANOVA deux voies.

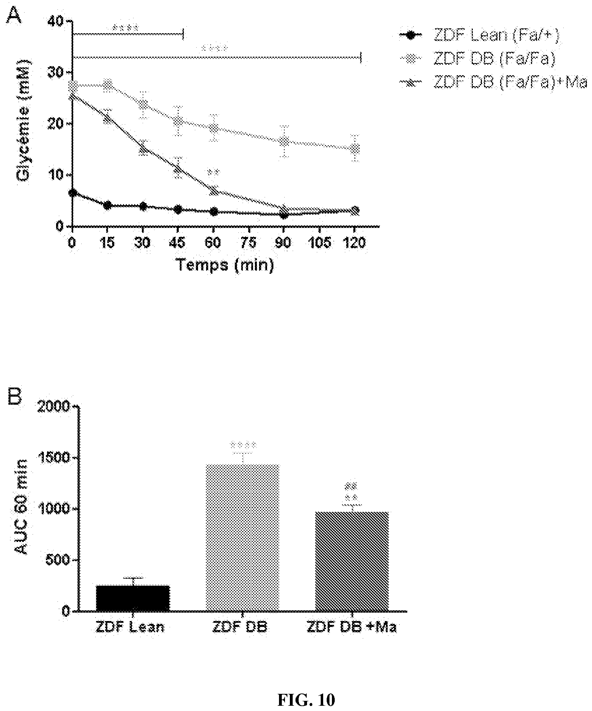

La **Figure 10** montre les résultats du test de sensibilité à l'insuline (1 U/kg) réalisé après 8 semaines du protocole d'étude dans les différents groupes de rats tels que définis dans la légende de la **Figure 8**.

**(A)** Graphique illustrant l'évolution de la glycémie au cours du test. Les deux groupes de rats diabétiques ZDF DB et ZDF DB+MA sont hyperglycémiques par rapport au groupe ZDF lean témoins durant les 45 premières minutes suivant l'injection d'insuline. Le groupe ZDF DB non traité est insulino-résistant tout au long du test comparé au groupe ZDF témoin alors que dans le groupe traité par MA (ZDF DB+MA), la résistance à l'insuline disparaît progressivement (amélioration significative dès 60 minutes, pour devenir non significative comparée au groupe témoin ZDF lean). Dans l'intervalle entre 75 et 120 minutes, la sensibilité à l'insuline devient identique entre les groupes ZDF DB+MA et ZDF lean, permettant ainsi une restauration complète de la glycémie en réponse à l'insuline exogène.

**** $p < 0,001$ : ZDF DB *vs*. ZDF lean
** $p < 0,01$ et **** $p < 0,001$ : ZDF DB+MA vs. ZDF lean

Test ANOVA deux voies.

**(B)** Aire sous la courbe intégrant la glycémie au cours des 60 premières minutes du test. Les rats ZDF DB+MA ont une excursion glycémique plus faible que les rats ZDF DB non traités mais plus élevée que les rats ZDF lean.

\*\* $p<0,01$ : ZDF DB+MA *vs*. ZDF lean
\*\*\*\* $p<0,0001$ : ZDF DB *vs*. ZDF lean
## $p<0,01$ : ZDF DB+MA *vs*. ZDF DB
Test ANOVA deux voies.

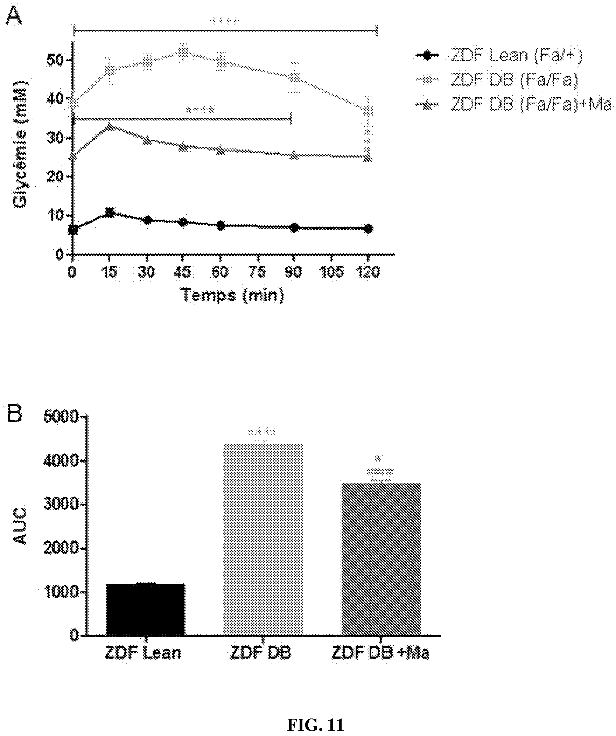

La **Figure 11** montre les résultats du test de tolérance au glucose (2 g/kg) réalisé après 8 semaines du protocole d'étude dans les différents groupes de rats tels que définis dans la légende de la **Figure 8**.

**(A)** Graphique illustrant l'évolution de la glycémie au cours du test. La glycémie est significativement plus élevée chez les rats ZDF DB que chez les rats ZDF+MA.

\*\* $p<0,01$ et \*\*\*\* $p<0,001$ : ZDF DB+MA vs. ZDF DB
\*\*\*\* $p<0,001$ : ZDF DB *vs*. ZDF lean
Test ANOVA deux voies.

**(B)** Histogrammes illustrant l'aire intégrée sous les courbes d'évolution de la glycémie en fonction du temps dans l'intervalle 0-60 minutes lors du test.

\* $p<0,05$ : ZDF DB+MA vs. ZDF-DB
\*\*\*\* $p<0,001$ : ZDF DB *vs*. ZDF lean
#### $p< 0,001$ : ZDF DB+MA vs. ZDF lean
Test ANOVA deux voies.

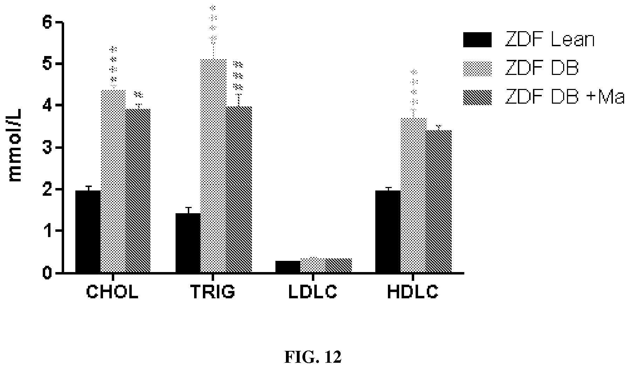

La **Figure 12** montre sous forme d'histogramme le bilan lipidique (cholestérol total et triglycérides) dans les 3 groupes de rats tels que définis dans la légende de la **Figure 8**. Les taux de cholestérol total (CHOL), de triglycérides (TRIG), de cholestérol des lipoprotéines de faible densité (en anglais, *low density lipoprotein cholestérol - LDLC)* et de cholestérol des lipoprotéines de haute densité (en anglais *High density lipoprotein cholestérol - HDLC*) sont indiqués.

\*\*\*\* $p<0,0001$ : ZDF DB *vs*. ZDF lean
# $p<0,05$ et ### $p<0,001$ : ZDF DB+MA *vs*. ZDF DB
Test ANOVA deux voies.

## EXEMPLES

La présente invention se comprendra mieux à la lecture des exemples suivants qui illustrent non-limitativement l'invention.

### Exemple 1 : Récolte et préparation d'extraits de *Moricandia arvensis* et *d'Astragalus armatus*

Un extrait de *Moricandia arvensis* a été préparé par décoction, en plongeant 100 g de feuilles séchées et réduites en poudre dans 1 litre d'eau bouillante pendant 15 à 20 minutes. Après filtration de la décoction, l'extrait a été lyophilisé. Le lyophilisat a ensuite été conservé à 4°C.
Un extrait d'*Astragalus armatus* a été préparé par macération, en plongeant 100 g de racines séchées et réduites en poudre dans 1 litre de méthanol pendant 15 jours sous agitation. Après filtration du macérat, le méthanol a été évaporé sous pression réduite avec un évaporateur rotatif à 40°C jusqu'à obtenir un extrait sec (poudre). L'extrait sec a ensuite été conservé à 4°C.

**Exemple 2 : Effets pharmacologiques des extraits de *Moricandia arvensis* et *d'Astragalus armatus* dans un modèle de développement du diabète de type 2**

Matériels et méthodes

*Animaux et protocole d'étude*

Des rats Wistar (Charles River laboratories, USA) ont été répartis en 3 groupes pour un protocole d'étude d'une durée de 26 jours :

- les rats du groupe 1 ont suivi un régime alimentaire standard (laboratoires SAFE, France) ;
- les rats du groupe 2 ont été nourris avec un régime HFHS (« High Fat High Sucrose » - référence H235, laboratoires SAFE, France) qui conduit aux symptômes du diabète de type 2 ;
- les rats du groupe 3 ont été nourris avec un régime HFHS et ont été traités chaque matin avec 250 mg/kg par gavage avec un extrait de *Moricandia arvensis* (HFHS - MA) ou d'*Astragalus armatus* (HFHS - AA) préparé comme décrit dans l'Exemple 1 ci-dessus.

La glycémie a été mesurée au début du protocole (jour 0), à la fin de la semaine 1 (jour 7), à la fin de la semaine 2 (jour 14) et la fin de la semaine 3 (jour 21). Plusieurs tests ont ensuite été réalisés dans une période de 5 jours à la fin du protocole (jours 22 à 26 - intervalle de repos de 2 jours entre chacun des tests). L'insulinémie basale a été mesurée. Un test de tolérance au glucose, OGTT (en anglais, *Oral Glucose Tolérance Test* - hyperglycémie provoquée par voie orale - 2 g/kg) a été réalisé. La glycémie et l'insulinémie ont été mesurées au cours de ce test. Un test de sensibilité à l'insuline (voie intra-péritonéale) (1 U/kg) a également été réalisé. La composition corporelle a été analysée par imagerie par résonance magnétique (EchoMRI) pour les groupes 1 et 2, et pour les animaux du groupe 3 traités avec un extrait de *Moricandia arvensis* au jour 26 du protocole d'étude, après la fin des tests. Les rats ont été pesés aux jours 0, 5, 9, 15, 18, 23, 25 et 26 du protocole d'étude.

*Analyse statistique*

Les valeurs des moyennes $\pm$ écart standard à la moyenne (esm), pendant et/ou à l'issue du traitement, sont présentées. L'effectif est de n=8 rats/groupe, sauf pour la mesure de la glycémie et de l'insulinémie basales pour laquelle n=9 à 11. Les calculs statistiques pour évaluer les différences entre les groupes (seuil de significativité : $p<0,05$) ont été réalisés par une analyse de variance ANOVA 2 voies (effet temps/traitement). Les analyses statistiques ont été réalisées à l'aide du logiciel Prism. Les différences significatives sont représentées sur les figures.

Résultats

Le traitement avec un extrait de *Moricandia arvensis* ou un extrait *d'Astragalus armatus* prévient l'hyperglycémie observée chez les animaux non-traités nourris avec le régime HFHS (**Figure 1** et **Tableau 1**).

**Tableau 1** : Glycémie à la fin de la semaine 3 (jour 21) pour les différents groupes.

| Groupe | Glycémie (mM $\pm$ esm) | Glycémie (g/L $\pm$ esm) |
|---|---|---|
| STD | 6,17 $\pm$ 0,17 | 1,10 $\pm$ 0,03 |
| HFHS | 7,98 $\pm$ 0,25 | 1,43 $\pm$ 0,04 |
| HFHS-MA | 6,52 $\pm$0,16 | 1,17 $\pm$ 0,03 |
| HFHS-AA | 6,16 $\pm$ 0,15 | 1,10 $\pm$ 0,03 |

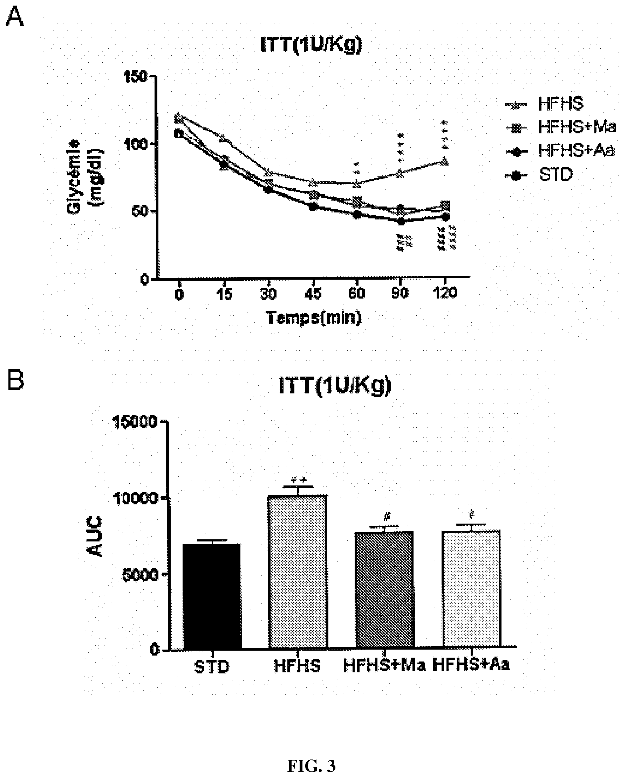

La mesure de l'insulinémie basale à la fin du protocole (**Figure 2** et **Tableau 2)** et les résultats du test de sensibilité à l'insuline **(Figures 3A** et **3B ; Tableaux 3 et 4)** montrent que le traitement avec un extrait de *Moricandia arvensis* ou un extrait *d'Astragalus armatus* prévient le développement d'une résistance à l'insuline, observée chez les animaux non-traités nourris avec le régime HFHS.

**Tableau 2** : Insulinémie basale à la fin du protocole pour les différents groupes.

| Groupe | Insulinémie (µU/mL $\pm$ esm) |
|---|---|
| STD | 52,0 $\pm$ 0,16 |
| HFHS | 62,2 $\pm$ 2,00 |
| HFHS-MA | 53,4 $\pm$ 2,6 |
| HFHS-AA | 42,7 $\pm$ 4,4 |

**Tableau 3 :** Glycémie à 120 minutes après l'injection d'insuline durant le test de sensibilité à l'insuline pour les différents groupes.

| Groupe | Glycémie (µU/mL $\pm$ esm) |
|---|---|
| STD | 52,0 $\pm$ 0,16 |
| HFHS | 62,2 $\pm$ 2,00 |
| HFHS-MA | 53,4 $\pm$ 2,6 |
| HFHS-AA | 42,7 $\pm$ 4,4 |

**Tableau 4 :** Aire sous les courbes d'évolution de la glycémie au cours du test de sensibilité à l'insuline.

| Groupe | Aire sous la courbe (unité arbitraire de surface $\pm$ esm) |
|---|---|
| STD | 6951 $\pm$ 351 |
| HFHS | 9965 $\pm$ 654 |
| HFHS-MA | 7634 $\pm$ 375 |
| HFHS-AA | 7479 $\pm$ 493 |

Le test de tolérance au glucose montre que le traitement avec un extrait de *Moricandia arvensis* ou un extrait d'*Astragalus armatus* prévient le développement d'une intolérance au glucose, observée chez les animaux non traités nourris avec le régime HFHS (**Figures 4A** et **4B** ; **Tableaux 5** et **6**). La mesure de l'insulinémie au cours du test de tolérance au glucose montre que le niveau d'insuline ne change pas chez les animaux du groupe 3 traité avec un extrait de *Moricandia arvensis* (**Figure 4C** ; **Tableau 7**). Cette observation suggère que l'effet de l'extrait de *Moricandia arvensis* sur la glycémie est médié par la préservation de la sensibilité à l'insuline. Chez les animaux traités avec un extrait d'*Astragalus armatus*, l'insulinémie est équivalente à celle mesurée dans le groupe contrôle, nourris avec un régime standard (**Figure 4C**). Cette observation indique que le traitement avec un extrait d'*Astragalus armatus*, outre son effet sur la sensibilité à l'insuline, prévient également la diminution de la capacité de sécrétion d'insuline observée chez les animaux non traités nourris avec le régime HFHS.

**Tableau 5** : Glycémie au cours du test de tolérance au glucose.

| Groupe | Glycémie (mg/dL $\pm$ esm) | | | | |
|---|---|---|---|---|---|
| Temps | 15 minutes | 45 minutes | 60 minutes | 90 minutes | 120 minutes |
| STD | - | 144,4 $\pm$ 5,5 | 141,8 $\pm$ 3,9 | 135,2 $\pm$ 1,8 | - |
| HFHS | 180,3 $\pm$ 5,9 | 173,9 $\pm$3,5 | 175,9 $\pm$ 5,1 | 160,0 $\pm$ 3,1 | 143,5 $\pm$ 4,9 |
| HFHS-MA | 154,4 $\pm$ 5,5 | 150,3 $\pm$ 6,0 | 146,0 $\pm$ 4,7 | 129,3 $\pm$ 4,9 | 118,0 $\pm$ 3,1 |
| HFHS-AA | - | 150,6 $\pm$ 4,5 | 138,3 $\pm$ 3,9 | 128,1 $\pm$ 3,8 | 116,5 $\pm$ 1,6 |

**Tableau 6** : Aire sous les courbes d'évolution de la glycémie au cours du test de tolérance au glucose dans l'intervalle de temps 0-60 minutes.

| Groupe | Aire sous la courbe (unité arbitraire de surface ± esm) |
|---|---|
| STD | 8882 ± 273 |
| HFHS | 10100 ± 92 |
| HFHS-MA | 8686 ± 284 |
| HFHS-AA | 8899 ± 230 |

**Tableau 7** : Insulinémie au cours du test de tolérance au glucose au temps +15 minutes.

| Groupe | Insulinémie ($\mu$U/mL ± esm) |
|---|---|
| STD | 67,2 ± 5,1 |
| HFHS | 46,3 ± 5,1 |
| HFHS-MA | 45,1 ± 5,1 |
| HFHS-AA | 74,4 ± 4,5 |

L'analyse de la composition corporelle montre que le traitement avec un extrait de *Moricandia arvensis* prévient l'augmentation de la proportion de masse grasse (et la diminution de la proportion de masse maigre associée) observée chez les animaux non traités nourris avec le régime HFHS **(Figures 5A** et **5B** ; **Tableau 8)**. Cette observation indique un mécanisme potentiel lié au développement de la masse grasse sous-jacent à la préservation de la sensibilité à l'insuline chez les animaux traités avec un extrait de *Moricandia arvensis.*

**Tableau 8** : Analyse de la composition corporelle.

| Groupe | Masse grasse (% du poids total sans eau ± esm) | Masse maigre (% du poids total sans eau ± esm) |
|---|---|---|
| STD | 13,7 ± 0,7 | 72,5 ± 0,7 |
| HFHS | 20,0 ± 1,1 | 66,9 ± 1,0 |
| HFHS-MA | 16,3 ± 0,9 | 71,7 ± 1,9 |

La pesée des animaux dans les 3 groupes au cours du protocole d'étude montre que le traitement avec un extrait de *Moricandia arvensis* ou un extrait d'*Astragalus armatus* prévient la prise de poids observée chez les animaux non traités nourris avec le régime HFHS **(Figure 6** et **Tableau 9)**.

**Tableau 9** : Évolution de la différence de poids aux jours 9, 18, 23 et 26.

| Groupe | Différence de poids (g ± esm) | | |
|---|---|---|---|
| Jour | 9 | 18 | 23 |
| STD | 26,8 ± 1,7 | 40,4 ± 2,2 | 65,1 ± 3,0 |
| HFHS | 45,5 ± 5,0 | 81,0 ± 5,7 | 101,6 ± 7,8 |
| HFHS-MA | 29,6 ± 3,3 | 52,1 ± 4,4 | 68,4 ± 7,0 |
| HFHS-AA | 28,4 ± 1,8 | 52,9 ± 2,7 | 79,0 ± 2,9 |

Conclusion

Ces résultats, pris dans leur ensemble, montrent que le traitement avec un extrait de *Moricandia arvensis* ou un extrait d'*Astragalus armatus* prévient l'hyperglycémie, la résistance à l'insuline, l'intolérance au glucose et la prise de poids observée dans un modèle développant les symptômes du diabète de type 2.

Dans le cas du traitement avec un extrait d'*Astraglalus armatus*, il apparait que celui-ci préserve également la capacité de sécrétion d'insuline, diminuée lorsque les animaux sont nourris avec le régime HFHS.

**Exemple 3 : Influence des extraits de *Moricandia arvensis* et *d'Astragalus armatus* sur la capacité de sécrétion d'insuline par des îlots pancréatiques de rats en culture primaire**

Matériels et méthodes

Des îlots pancréatiques de souris C57Bl6 ont été isolés par taille, répartis en puits de culture (en veillant à respecter une distribution équivalente dans chaque puits en termes de taille) et mis en culture en condition normale (contrôle) ou en condition lipotoxique en présence de palmitate :

- [Contrôle] : milieu de culture seul ;
- [Palmitate] : milieu de culture + 0,5 mM palmitate (acide gras saturé mimant les conditions diabétiques - DT2) ;
- [Palmitate+FM50] : milieu de culture + 0,5 mM palmitate + 50 $\mu$g/mL d'extrait de *Moricandia arvensis* ;
- [Palmitate+FM100] : milieu de culture + 0,5 mM palmitate + 100 $\mu$g/mL d'extrait de *Moricandia arvensis* ;
- [Palmitate+RA50] : milieu de culture + 0,5 mM palmitate + 50 $\mu$g/mL d'extrait *d'Astragalus armatus* ;
- [Palmitate+RA100] : milieu de culture + 0,5 mM palmitate + 100 $\mu$g/mL d'extrait *d'Astragalus armatus* ;

Les extraits de *Moricandia arvensis* et *d'Astragalus armatus* ont été préparés comme décrit dans l'Exemple 1 ci-dessus.

Après 72 heures de culture, la capacité de sécrétion d'insuline des îlots pancréatiques a été testée en mesurant la concentration d'insuline avant et après stimulation par une augmentation de la concentration de glucose dans le milieu de 2,8 mM (condition basale) à 16,7 mM (condition stimulée).

Résultats

La capacité de sécrétion basale et après stimulation des îlots pancréatiques, diminuée en présence de palmitate, est en grande partie préservée par la présence d'extrait de *Moricandia arvensis* ou d'extrait *d'Astragalus armatus* dans le milieu **(Figure 7** et **Tableau 10)**.

**Tableau 10** : Sécrétion d'insuline par les îlots pancréatiques.

| Condition de culture | Sécrétion d'insuline (ng/îlot/h ± esm) | |
|---|---|---|
| Simulation | Non | Oui |
| Contrôle | 0,126 ± 0,010 | 2,869 ± 0,066 |
| Palmitate | 0,084 ± 0,016 | 1,845 ± 0,0183 |
| Palmitate + FM50 | 0,118 ± 0,014 | 2,525 ± 0,280 |
| Palmitate + FM100 | 0,141 ± 0,002 | 2,430 ± 0,255 |
| Palmitate + RA50 | 0,117 ± 0,016 | 2,279 ± 0,163 |
| Palmitate + RA100 | 0,126 ± 0,011 | 2,349 ± 0,324 |

Conclusion

Ces résultats indiquent un effet protecteur des extraits de *Moricandia arvensis* et d'*Astragalus armatus* de la capacité de sécrétion d'insuline par les cellules pancréatiques, et ainsi de l'installation d'un diabète de type 2 en cas de régime riche en graisse et sucres.

**Exemple 4 : Effets curatifs de MA dans un modèle génétique de diabète de type 2 (rat Zücker ZDF)**

Matériels et méthodes

*Animaux et protocole d'étude*

Les effets anti-diabétiques des extraits MA ont été testés dans un modèle de DT2 sévère : le rat ZDF (Zücker diabetic fatty rat), modèle génétique qui présente une mutation récessive du récepteur à la leptine (seuls les animaux portant la mutation sur les deux allèles développent une obésité et un DT2). Cette résistance totale à la leptine entraîne une hyperphagie, une obésité massive et une résistance à l'insuline qui sont irréversibles : le diabète s'installe et ne cesse de s'accroître avec l'âge. En premier lieu, une hyperinsulinémie se développe au sevrage (à 3 semaines d'âge) pour compenser la résistance des tissus, mais le pancréas endocrine s'épuise et les animaux deviennent rapidement diabétiques, car leur sécrétion d'insuline décline et finalement s'épuise.

C'est dans ce modèle que les extraits de MA ont été testés, dans les mêmes conditions que pour les effets préventifs des Exemples précédents (250 mg/kg, par gavage journalier, pendant 8 à 13 semaines).

Les animaux âgés de 6 semaines ont été répartis en 3 groupes pour un protocole d'étude d'une durée de 13 semaines :

- ZDF Lean (*fa*/+): contrôle hétérozygote pour la mutation récessive ou homozygotes pour l'allèle sauvage du récepteur à la leptine. C'est le groupe normal de référence.
- ZDF DB (*fa*/*fa*) : homozygotes pour la mutation du récepteur à la leptine. Les animaux sont obèses, insulino-résistants puis diabétiques de façon irréversible.
- ZDF DB (*fa*/*fa*) + MA : homozygotes pour la mutation du récepteur à la leptine traité avec 250 mg/kg par gavage avec un extrait de *Moricandia arvensis* préparé comme décrit dans l'Exemple 1 ci-dessus.

Les animaux sont sous un régime normal (SAFE). Les différentes mesures et tests ont été réalisés au cours des 8 semaines à 13 semaines.

*Analyse statistique*

L'effectif par groupe est n=6. Les résultats sont présentés sous la forme de moyenne $\pm$ écart standard à la moyenne (esm). Les analyses statistiques sont des analyses de variance (ANOVA), avec un seuil de significativité à $p < 0,05$. Les différences significatives sont représentées sur les figures.

Résultats

Le traitement avec un extrait de *Moricandia arvensis* diminue l'hyperglycémie observée à partir de la semaine 3 chez les homozygotes pour la mutation du récepteur à la leptine non traités **(Figure 8** et **Tableau 11)**.

**Tableau 11** : Glycémie à la semaine 11 du protocole d'étude.

| Groupe | Glycémie (mg/dL $\pm$ esm) |
|---|---|
| ZDF lean | 116,5 $\pm$ 3,6 |
| ZDF DB | 525,8 $\pm$ 20,2 |
| ZDF DB + MA | 454,1 $\pm$ 30,3 |

La mesure de l'insulinémie basale au cours des 8 premières semaines du protocole **(Figure 9** et **Tableau 12)** et les résultats du test de sensibilité à l'insuline **(Figure 10A** et **10B ; Tableau 13)** montrent que le traitement avec un extrait de *Moricandia arvensis* soigne la résistance à l'insuline observée chez les homozygotes pour la mutation du récepteur à la leptine non traités.

**Table 12 :** Aire sous les courbes d'évolution de l'insulinémie basale durant les 8 premières semaines du protocole.

| Groupe | Aire sous la courbe (unité arbitraire de surface $\pm$ esm) |
|---|---|
| ZDF lean | 8,11 $\pm$ 0,17 |
| ZDF DB | 13,76 $\pm$ 0,76 |
| ZDF DB + MA | 12,03 $\pm$ 0,92 |

**Tableau 13** : Aire sous les courbes d'évolution de la glycémie au cours des premières 60 minutes du test de sensibilité à l'insuline.

| Groupe | Aire sous la courbe (unité arbitraire de surface ± esm) |
|---|---|
| ZDF lean | 243,5 ± 33,9 |
| ZDF DB | 1424,0 ± 119,9 |
| ZDF DB + MA | 964,9 ± 71,4 |

Le test de tolérance au glucose montre que le traitement avec un extrait de *Moricandia arvensis* permet de diminuer l'intolérance au glucose observée chez les homozygotes pour la mutation du récepteur à la leptine non traités **(Figures 11A** et **11B** ; **Tableau 14)**.

**Tableau 14** : Aire sous les courbes d'évolution de la glycémie au cours des premières 60 minutes du test de tolérance au glucose.

| Groupe | Aire sous la courbe (unité arbitraire de surface ± esm) |
|---|---|
| ZDF lean | 1183,8 ± 8,5 |
| ZDF DB | 4371,0 ± 93,2 |
| ZDF DB + MA | 3472,8 ± 67,6 |

Le bilan lipidique montre que le traitement avec un extrait de *Moricandia arvensis* permet de diminuer le taux de cholestérol, de triglycérides et de cholestérol des lipoprotéines de haute densité (en anglais *high density lipoprotein cholestérol - HDLC*) anormalement élevé chez les homozygotes pour la mutation du récepteur à la leptine non traités **(Figure 12** et **Tableau 15)**. Compte-tenu du rôle inflammatoire des lipides et du rôle de l'inflammation dans l'installation de la résistance à l'insuline, un des effets potentiels de *Moricandia arvensis* pourrait passer par l'amélioration du bilan lipidique.

**Table 15** : Bilan lipidique.

| Groupe | Cholestérol total (mmol/L ± esm) | Triglycérides (mmol/L ± esm) |
|---|---|---|
| ZDF lean | 2,0 ± 0,1 | 1,4 ± 0,1 |
| ZDF DB | 4,4 ± 0,1 | 5,1 ± 0,4 |
| ZDF DB + MA | 3,9 ± 0,1 | 4,0 ± 0,3 |

Conclusions

Ces résultats, pris dans leur ensemble, montrent que l'administration d'un extrait de *Moricandia arvensis* permet de traiter, de manière curative, le diabète de type 2 dans ce modèle pré-clinique.

**Exemple 5 : Effets pharmacologiques d'une combinaison d'extraits de *Moricandia arvensis* et d'*Astragalus armatus* dans un modèle de développement du diabète de type 2**

Matériels et méthodes

*Animaux et protocole d'étude*

Des rats Wistar (Charles River laboratories, USA) sont répartis en 3 groupes pour un protocole d'étude d'une durée de 26 jours :

- les rats du groupe 1 suivent un régime alimentaire standard (laboratoires SAFE, France) ;
- les rats du groupe 2 sont nourris avec un régime HFHS (« High Fat High Sucrose »

  - référence H235, laboratoires SAFE, France) qui conduit aux symptômes du diabète de type 2 ;

- les rats du groupe 3 sont nourris avec un régime HFHS et ont été traité chaque matin avec 250 mg/kg par gavage avec d'une combinaison d'extraits de *Moricandia arvensis* et d'*Astragalus armatus* (HFHS - MA+AA).

La glycémie est mesurée au début du protocole (jour 0), à la fin de la semaine 1 (jour 7), à la fin de la semaine 2 (jour 14) et la fin de la semaine 3 (jour 21). Plusieurs tests sont ensuite réalisés dans une période de 5 jours à la fin du protocole (jours 22 à 26 - intervalle de repos de 2 jours entre chacun des tests). L'insulinémie basale est mesurée. Un test de tolérance au glucose, OGTT (en anglais, *Oral Glucose Tolérance Test* - hyperglycémie provoquée par voie orale - 2 g/kg) est réalisé. La glycémie et l'insulinémie sont mesurées au cours de ce test. Un test de sensibilité à l'insuline (voie intra-péritonéale) (1 U/kg) est également réalisé. La composition corporelle est analysée par imagerie par résonance magnétique (EchoMRI) pour les groupes 1 et 2, et pour les animaux du groupe 3 traités avec une combinaison d'extraits de *Moricandia arvensis* et d'*Astragalus armatus* au jour 26 du protocole d'étude, après la fin des tests. Les rats sont pesés aux jours 0, 5, 9, 15, 18, 23, 25 et 26 du protocole d'étude.

**Exemple 6 : Influence d'une combinaison d'extraits de *Moricandia arvensis* et *d'Astragalus armatus* sur la capacité de sécrétion d'insuline par des îlots pancréatiques de rats en culture primaire**

Matériels et méthodes

Des îlots pancréatiques de souris C57Bl6 sont isolés par taille, répartis en puits de culture (en veillant à respecter une distribution équivalente dans chaque puits en terme de taille) et mis en culture en condition normale (contrôle) ou en condition lipotoxique en présence de palmitate :

- [Contrôle] : milieu de culture seul ;
- [Palmitate] : milieu de culture + 0,5 mM palmitate (acide gras saturé mimant les conditions diabétiques - DT2) ;
- [Palmitate+FM25/RA25] : milieu de culture + 0,5 mM palmitate + 25 $\mu$g/mL d'extrait de *Moricandia arvensis* + 25 $\mu$g/mL d'extrait d'*Astragalus armatus* ;
- [Palmitate+FM50/RA50] : milieu de culture + 0,5 mM palmitate + 50 $\mu$g/mL d'extrait de *Moricandia arvensis* + 50 $\mu$g/mL d'extrait d'*Astragalus armatus* ;
- [Palmitate+FM100/RA100] : milieu de culture + 0,5 mM palmitate + 100 $\mu$g/mL d'extrait de *Moricandia arvensis* + 100 $\mu$g/mL d'extrait d'*Astragalus armatus.*

Les extraits de *Moricandia arvensis* et d'*Astragalus armatus* sont préparés comme décrit dans l'Exemple 1 ci-dessus.

Après 72 heures de culture, la capacité de sécrétion d'insuline des îlots pancréatiques est testée en mesurant la concentration d'insuline avant et après stimulation par une augmentation de la concentration de glucose dans le milieu de 2,8 mM (condition basale) à 16,7 mM (condition stimulée).

**Exemple 7 : Effets curatifs d'une combinaison d'extraits de *Moricandia arvensis* et *d'Astragalus armatus* dans un modèle génétique de diabète de type 2 (rat Zücker ZDF)**

Matériels et méthodes

*Animaux et protocole d'étude*

Les effets anti-diabétiques d'une combinaison d'extraits de *Moricandia arvensis* et d'*Astragalus armatus* sont testés dans un modèle de DT2 sévère : le rat ZDF (Zücker diabetic fatty rat), modèle génétique qui présente une mutation récessive du récepteur à la leptine (seuls les animaux portant la mutation sur les deux allèles développent une obésité et un DT2). Cette résistance totale à la leptine entraîne une hyperphagie, une obésité massive et une résistance à l'insuline qui sont irréversibles : le diabète s'installe et ne cesse de s'accroître avec l'âge. En premier lieu, une hyperinsulinémie se développe au sevrage (à 3 semaines d'âge) pour compenser la résistance des tissus, mais le pancréas endocrine s'épuise et les animaux deviennent rapidement diabétiques, car leur sécrétion d'insuline décline et finalement s'épuise.

C'est dans ce modèle qu'une combinaison d'extraits de *Moricandia arvensis* et d'*Astragalus armatus* est testée, dans les mêmes conditions que pour les effets préventifs des exemples précédents (250 mg/kg, par gavage journalier, pendant 8 à 13 semaines).

Les animaux âgés de 6 semaines sont répartis en 3 groupes pour un protocole d'étude d'une durée de 13 semaines :

- ZDF Lean (*fa*/+): contrôle hétérozygote pour la mutation récessive ou homozygotes pour l'allèle sauvage du récepteur à la leptine. C'est le groupe normal de référence.
- ZDF DB (*fa*/*fa*) : homozygotes pour la mutation du récepteur à la leptine. Les animaux sont obèses, insulino-résistants puis diabétiques de façon irréversible.
- ZDF DB (*fa*/*fa*) + MA/AA : homozygotes pour la mutation du récepteur à la leptine traité avec 250 mg/kg par gavage avec une combinaison d'extraits de *Moricandia arvensis* et d'*Astragalus armatus* préparé comme décrit dans l'Exemple 1 ci-dessus.

Les animaux sont sous un régime normal (SAFE). Les différentes mesures et tests sont réalisés au cours des 8 semaines à 13 semaines.

**Revendications**

1. Une composition comprenant une décoction de feuilles de *Moricandia arvensis*, optionnellement lyophilisée, pour son utilisation dans la prévention et/ou le traitement d'une maladie métabolique sélectionnée dans le groupe comprenant le diabète, la résistance à l'insuline, l'intolérance au glucose, et l'hyperglycémie.

2. La composition pour son utilisation selon la revendication **1**, dans laquelle la maladie métabolique est sélectionnée dans le groupe comprenant le diabète de type 2, la résistance à l'insuline, l'intolérance au glucose et l'hyperglycémie.

3. La composition pour son utilisation selon la revendication **1** ou **2**, dans laquelle la maladie métabolique est le diabète de type 2.

4. La composition pour son utilisation selon l'une quelconque des revendications **1** à **3**, comprenant en outre un extrait d'*Astragalus armatus.*

5. La composition pour son utilisation selon la revendication **4**, dans laquelle l'extrait d'*Astragalus armatus* est une décoction et/ou une macération d'*Astragalus armatus.*

6. La composition pour son utilisation selon la revendication **4** ou **5**, dans laquelle l'extrait *d'Astragalus armatus* est une macération de racines *d'Astragalus armatus*, de préférence une macération méthanolique de racines d'*Astragalus armatus*, optionnellement lyophilisé.

7. Une méthode non-thérapeutique de :

   - prévention de la prise de poids chez un sujet, de préférence de prévention de la prise de masse grasse chez un sujet ;
   - contrôle de la prise de poids chez un sujet, de préférence de contrôle de la prise de masse grasse chez un sujet ; ou
   - stimulation de la perte de poids chez un sujet, de préférence de stimulation de la perte de masse grasse chez un sujet ;

   comprenant l'administration audit sujet d'un alicament comprenant une décoction de feuilles de *Moricandia arvensis*, optionnellement lyophilisée.

8. La méthode non-thérapeutique selon la revendication **7**, dans laquelle le sujet n'est pas affecté par et/ou n'a pas été diagnostiqué avec une maladie métabolique telle que définie à la revendication **1**.

9. La méthode non-thérapeutique selon la revendication **7** ou **8**, dans laquelle l'alicament comprend en outre un extrait d'*Astragalus armatus*, optionnellement lyophilisé.

10. La méthode non-thérapeutique selon l'une quelconque des revendications **7** à **9**, dans laquelle l'alicament comprend en outre une décoction et/ou une macération *d'Astragalus armatus*, optionnellement lyophilisé.

11. La méthode non-thérapeutique selon l'une quelconque des revendications **7** à **10**, dans laquelle l'alicament comprend en outre une macération de racines *d'Astragalus armatus*, de préférence une macération méthanolique de racines d'*Astragalus armatus*, optionnellement lyophilisé.

**Patentansprüche**

1. Zusammensetzung, die ein Dekokt aus *Moricandia arvensis*-Blättern umfasst, die gegebenenfalls lyophilisiert ist, zur Verwendung bei der Vorbeugung und/oder Behandlung einer Stoffwechselerkrankung, die aus der Gruppe ausgewählt ist, die Diabetes, Insulinresistenz, Glukoseintoleranz und Hyperglykämie umfasst.

2. Zusammensetzung zur Verwendung nach Anspruch 1, wobei die Stoffwechselerkrankung aus der Gruppe ausgewählt ist, die Typ-2-Diabetes, Insulinresistenz, Glukoseintoleranz und Hyperglykämie umfasst.

3. Zusammensetzung zur Verwendung nach Anspruch 1 oder 2, wobei die Stoffwechselerkrankung Typ-2-Diabetes ist.

4. Zusammensetzung zur Verwendung nach einem der Ansprüche 1 bis 3, die ferner einen Extrakt aus *Astragalus armatus* umfasst.

5. Zusammensetzung zur Verwendung nach Anspruch 4, wobei der Extrakt aus *Astragalus armatus* ein Dekokt und/oder eine Mazeration von *Astragalus armatus* ist.

6. Zusammensetzung zur Verwendung nach Anspruch 4 oder 5, wobei der Extrakt aus *Astragalus armatus* eine Mazeration von *Astragalus armatus-Wurzeln*, vorzugsweise eine methanolische Mazeration von *Astragalus armatus-Wurzeln*, ist, der gegebenenfalls lyophilisiert ist.

7. Nicht therapeutisches Verfahren zum:

   - Verhindern einer Gewichtszunahme bei einer Person, vorzugsweise Verhindern der Zunahme von Fettmasse bei einer Person;
   - Kontrollieren der Gewichtszunahme bei einer Person, vorzugsweise Kontrollieren der Zunahme von Fettmasse bei einer Person;
   - Stimulieren des Gewichtsverlusts bei einer Person, vorzugsweise Stimulieren des Verlusts von Fettmasse bei einer Person;

   umfassend das Verabreichen eines funktionellen Lebensmittels, das ein Dekokt aus *Moricandia arvensis*-Blättern umfasst und gegebenenfalls lyophilisiert ist, an die Person.

8. Nicht therapeutisches Verfahren nach Anspruch 7, wobei die Person nicht von einer Stoffwechselerkrankung, wie in Anspruch 1 definiert, betroffen ist und/oder mit einer solchen diagnostiziert wurde.

9. Nicht therapeutisches Verfahren nach Anspruch 7 oder 8, wobei das funktionelle Lebensmittel ferner einen Extrakt aus *Astragalus armatus* umfasst und gegebenenfalls lyophilisiert ist.

10. Nicht therapeutisches Verfahren nach einem der Ansprüche 7 bis 9, wobei das funktionelle Lebensmittel ferner ein Dekokt und/oder eine Mazeration von *Astragalus armatus* umfasst und gegebenenfalls lyophilisiert ist.

11. Nicht therapeutisches Verfahren nach einem der Ansprüche 7 bis 10, wobei das funktionelle Lebensmittel ferner eine Mazeration von *Astragalus armatus*-Wurzeln, vorzugsweise eine methanolische Mazeration von *Astragalus armatus*-Wurzeln, umfasst und gegebenenfalls lyophilisiert ist.


**Claims**

1. A composition comprising a decoction of leaves of *Moricandia arvensis,* optionally freeze-dried, for use for preventing and/or treating a metabolic disease selected from the group comprising diabetes, insulin resistance, glucose intolerance, and hyperglycemia.

2. The composition for use according to claim **1**, wherein the metabolic disease is selected from the group comprising type 2 diabetes, insulin resistance, glucose intolerance and hyperglycemia.

3. The composition for use according to claim **1** or **2**, wherein the metabolic disease is type 2 diabetes.

4. The composition for use according to any one of claims **1** to **3**, further comprising an extract of *Astragalus armatus*.

5. The composition for use according to claim **4**, in which the extract of *Astragalus armatus* is a decoction and/or a maceration of *Astragalus armatus.*

6. The composition for use according to claim **4** or **5**, in which the extract of *Astragalus armatus* is a maceration of roots of *Astragalus armatus*, preferably a methanolic maceration of roots of *Astragalus armatus*, optionally freeze-dried.

7. A non-therapeutic method of:

   - prevention of weight gain in a subject, preferably prevention of fat mass gain in a subj ect;
   - control of weight gain in a subject, preferably control of fat mass gain in a subject; or
   - stimulation of weight loss in a subject, preferably stimulation of fat mass loss in a subj ect;

   comprising the administration to said subject of a nutraceutical comprising a decoction of leaves of *Moricandia arvensis*, optionally freeze-dried.

8. The non-therapeutic method according to claim **7**, wherein the subject is not affected by and/or has not been diagnosed with a metabolic disease as defined in claim **1**.

9. The non-therapeutic method according to claim **7** or **8**, in which the nutraceutical further comprises an extract of *Astragalus armatus,* optionally freeze-dried.

10. The non-therapeutic method according to any one of claims **7** to **9**, in which the nutraceutical further comprises a decoction and/or maceration of *Astragalus armatus*, optionally freeze-dried.

11. The non-therapeutic method according to any one of claims **7** to **10**, in which the nutraceutical further comprises a maceration of *Astragalus armatus* roots, preferably a methanolic maceration of *Astragalus armatus* roots, optionally freeze-dried.

**FIG. 1**

**FIG. 2**

FIG. 3

A

B

C

FIG. 4

A

## masse grasse

B

## masse maigre

FIG. 5

FIG. 6

FIG. 7

FIG. 8

**Insulinémie**

FIG. 9

**FIG. 10**

FIG. 11

**FIG. 12**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 8946283 B

**Littérature non-brevet citée dans la description**

- **SKANDRANI et al.** *Food Chem Toxicol.,* 2010, vol. 48 (2), 710-5
- **SKANDRANI et al.** *Drug Chem Toxicol.,* 2007, vol. 30 (4), 361-82
- **BRAHAM et al.** *J Nat Prod.,* 2005, vol. 68 (4), 517-22
- Species 2000 & ITIS Catalogue of Life. 30 Juin 2018
- *Species 2000: Naturalis, Leiden, the Netherlands,* ISSN 2405-8858
- **FORD.** *Diabètes Care,* 2005, vol. 28 (7), 1769-78
- **BUGIANESI et al.** *Curr Pharm Des.,* 2010, vol. 16 (17), 1941-51
- **DIAMANTI-KANDARAKIS.** *Endocrine,* 2006, vol. 30 (1), 13-7
- **WATSON ; CRAFT.** *CNS Drugs.,* 2003, vol. 17 (1), 27-45
- **ARCIDIACONO et al.** *Exp Diabètes Res.,* 2012, vol. 2012, 789174